# EUROPEAN PATENT APPLICATION

(11) **EP 4 130 038 A1**
(43) Date of publication of application: **08.02.2023**
(21) Application number: 21306082.5
(22) Date of filing: 03.08.2021
(51) Int. Cl.: C07K 16/24, A61P 11/06, A61P 37/08, A61K 38/20, A61K 39/395

(54) **ANTI-IL-2 ANTIBODY, COMPLEX COMPRISING IT, AND USES THEREOF**

(71) Applicant: Institut national de recherche pour l'agriculture, l'alimentation et l'environnement, 75007 Paris (FR); Université de Nantes, 44035 Nantes (FR); Institut National de la Santé et de la Recherche Médicale, 75013 Paris (FR)
(72) Inventor: BOUCHAUD, Grégory, 44710 Port Saint Père (FR); TRANQUET, Olivier, 44240 La Chapelle sur Erdre (FR); MAGNAN, Antoine, 44000 Nantes (FR)
(74) Representative: Nony

(57) **Abstract**

The invention relates to an isolated anti-human IL-2 antibody, or an isolated antigen-binding portion thereof, as well as to an IL-2 complex comprising the anti-human IL-2 antibody, or the antigen-binding portion thereof, and a human IL-2, the antibody, or antigen-binding portion thereof, being non-covalently bound to the human IL-2. The invention further relates to their implementation in the prevention and/or treatment of respiratory and/or food allergies, or asthma, in a patient in need thereof and to a kit comprising the said antibody and IL-2.

## Description

### [TECHNICAL FIELD]

The present invention relates to the prevention and/or treatment of allergies, in particular respiratory and food allergies, as well as asthma. More particularly, the invention is concerned with the implementation of anti-IL-2 antibodies and isolated antigen-binding portion thereof, as well as to IL-2 complexes comprising (i) an anti-human IL-2 antibody, or the antigen-binding portion thereof, bound to a human IL-2, to induce regulatory T cells (Treg) *in vivo.*

### [TECHNICAL BACKGROUND]

Allergies are serious medical conditions with consequences ranging from non-life threatening responses that resolve over time to life threatening effects such as anaphylaxis. Allergic reactions can result from contact or exposure to a variety of products such as certain food items, insect venom, plant-derived material (e.g., pollen), chemicals, drugs/medications, and animal dander.

The prevalence of asthma and allergy, defined as immunologically mediated hypersensitivity, has been increasing for the past decades and is expected to keep growing. The World Health Organization estimates that asthma, which in more than 50% of adults and in at least 80% of affected children is allergic, affected nearly 262 million people in 2019 and caused 461 000 deaths, placing an enormous strain on health resources in many countries. In the USA alone, more than US$18 billion is spent globally each year on allergic rhinitis, including medications, time off work, and clinician consultations. Food allergies alone cost about US$25 billion each year to the USA alone. Certain environmental allergens, for example the food allergens from tree nuts and especially peanuts, have gained significant attention of the public because of coverage in the popular media. Notwithstanding the greater public awareness, as well as improved understanding of the pathophysiology of allergic responses in sensitized individuals, medical researchers have noted an increase of epidemic proportions in recent years in both morbidity and mortality in developing and developed countries.

Current treatment options for allergies include avoidance, pharmacological symptom treatment and prophylaxis using allergen-specific immunotherapies (AIT), also known as desensitization therapy. Unfortunately, these current treatment strategies are often inadequate, costly, impractical or involve significant risks. For example, avoidance of allergen is not always possible and can negatively impact on patient and caregiver quality of life. Immunotherapeutic approaches, on the other hand, also termed desensitization, involve deliberate administration of allergen to susceptible individuals and is therefore inherently risky with the potential for unwanted severe allergic reactions or anaphylaxis. It moreover usually takes years to establish an acceptable level of tolerance.

Accordingly, an unmet need exists in the art for novel therapeutic approaches that prevent or treat allergic responses and improve the safety and/or efficacy of immunotherapeutic treatment strategies.

Regulatory T cells (Treg) are known to play an important part in inhibiting harmful immunopathological responses directed against self or foreign antigens. As such, Tregs are known to protect against allergies.

In asthmatic patients, the lack of immune tolerance is associated with a decrease in Treg numbers in blood and bronchoalveolar lavage fluid (BALF), and these Tregs fail to suppress the proliferation and production of T helper 2 cells (Th2) and associated cytokines (Hartl D et al. The Journal of 432 allergy and clinical immunology. 2007;119(5): 1258-1266 and Mamessier E et al. Allergy. 2008;63(9):1202-1210). In allergic asthma, allergen-specific immunotherapy (AIT) can restore immune tolerance by inducing allergen-specific Tregs but requires 1 to 2 years to reach its full efficacy, with nonnegligible failure risks and side effects. Moreover, the risk of asthma exacerbation prevents AIT use in partially controlled or uncontrolled asthmatic patients (Passalacqua G, Canonica GW. Clinical and experimental allergy journal of the British Society for Allergy and Clinical Immunology. 2011;41(9):1247-1255; Shreffler WG et al. The Journal of allergy and clinical 451 immunology. 452 2009;123(1):43-52 e47).

Similar to asthma, food allergic patients also display a quantitative and/or qualitative defect in Tregs (Dang TD et al. Pediatric allergy and immunology : official publication of the European Society of Pediatric Allergy and Immunology. 2016;27(1):35-43). In food allergies, AIT is still under investigation despite promising results, but Bonnet et al. (Journal of immunology. 2016;197(1): 188-198) showed that Treg impairment can be restored under IL-2 low-dose therapy in OVA-induced food allergies in mice, suggesting that the IL-2/Treg dialogue could be a cornerstone in immune tolerance reestablishment.

Nevertheless, the question about IL-2 safety remains.

Indeed, IL-2 is well known to be critical for Treg development, activity and survival (Boyman et al. Current opinion in immunology. 2007;19(3):320-326; Malek TR et al. Journal of clinical immunology. 2008;28(6):635-639), but its use can also be harmful. IL-2-based therapy has already been proposed in metastatic melanoma and metastatic renal cell carcinoma, but its clinical application remains relatively restricted due to several shortcomings. Indeed, IL-2 is eliminated via the kidney, reducing its half-life in serum to several minutes. To achieve an optimal immunomodulatory effect, IL-2 should be repeatedly given at a high dose that results in severe toxicities, including vascular leak syndrome (VLS), lung edema and heart toxicities (Gately MK et al. Journal of immunology. 1988; 141 (1):189-200; Peace DJ, Cheever MA. The Journal of experimental medicine. 1989; 169(1): 161-173). Several studies have tried to improve the efficacy of IL-2 immunotherapy, notably via the combination of IL-2 with an anti-IL-2 antibody, enhancing the IL-2 half-life and biological activity (Letourneau S et al. Proceedings of the National Academy of Sciences of the United States of America. 2010;107(5):2171-2176).

It has previously been shown that some combinations of IL-2 with an anti-IL-2 antibody can selectively target CD4(+) and CD8(+) effector T cells or Tregs depending on the clone of the implemented anti-IL-2 antibody (Krieg C et al. Proceedings of the National Academy of Sciences of the United States of America. 2010;107(26):11906-11911). More particularly, Boyman O et al. (Science 311:1924-1927) previously demonstrated that when coupled with IL-2, some IL-2/IL-2 mAb complexes cause massive (>100-fold) expansion of CD8+ cells *in vivo,* whereas others selectively stimulate CD4+ T regs. Boyman *et al.* indicate that such opposed pro-inflammatory or anti-inflammatory activities of the complexes depend on the site of binding of the anti-IL-2 antibody to IL-2. Illustratively, Boyman *et al.* indicate that:
- using the JES6-1 anti-IL-2 antibody, JES6-1/IL-2 complexes were able to induce *in vivo* expansion of CD4⁺CD25⁺ Tregs cells due to JES6-1 mAb binding to an IL-2 site that is crucial for interaction with CD122 (IL-2R β-chain) but less crucial for binding to CD25;
- on the contrary, using the S4B6 antibody which binds to an IL-2 site that partly occludes binding to CD25 but does not impede binding to CD122, S4B6/IL-2 complexes caused considerable expansion of CD122^{hi} CD8⁺ cells.

Accordingly, Boyman *et al.* conclude that injecting IL-2/IL-2 mAb complexes with S4B6 might be clinically useful for tumor immunotherapy and for expanding T cell numbers after bone marrow transplantation. Conversely, IL-2/IL-2 mAb complexes with JES6-1 causing the expansion of CD4⁺ Tregs could be useful for treating autoimmune disease.

Moreover, Thornton A. et al. (Eur J Immunol. 2019 Mar; 49(3): 398-412) for example described that both mouse and human Foxp3⁺ Treg can be divided into two different major subpopulations composed of Foxp3⁺Helios⁺ (∼70%) and Foxp3⁺Helios⁻ (∼30%) cells. The Helios⁺ and Helios⁻ populations of Treg are distinct subpopulations that can be stimulated by different agents. Helios⁺ and Helios⁻ Treg subpopulations are phenotypically and functionally distinct and express dissimilar TCR repertoires, with Foxp3 expression being much more stable in Helios⁺ Treg. Helios⁺ Treg have in particular been demonstrated as having superior suppressive properties as compared to the Helios⁻ population. Accordingly, Thornton *et al.* conclude that stimulation of the Foxp3⁺Helios⁻ Treg population in man would clearly be an inappropriate population to use for cellular biotherapy or to expand *in vivo* with cytokines or other biologics as it would be poorly suppressive, lack stability, and be deficient in a TCR repertoire with self-antigen specificity.

There accordingly remains a need for the provision of novel therapeutic agents that prevent or treat allergic responses and asthma, in particular food allergies, respiratory allergies and asthma.

In particular, there remains a need for an improved and safer therapeutic strategy to prevent or treat such disorders in a patient in need thereof.

There further is a need for novel therapeutic agents able to efficiently induce Tregs in vivo without any side effects.

There is in particular a need for novel therapeutic agents able to *in vivo* efficiently and safely stimulate the increase of Treg numbers but also boost Treg functions, in particular to improve the suppressive function of the Tregs cells, more particularly through increasing Helios expression on Tregs cells.

There also remains a need to improve and shorten current desensitization therapies against a given allergen.

The present invention has for purpose to satisfy all or part of those needs.

### [SUMMARY]

The present invention relates to the following items:
**Item 1:** An isolated anti-human IL-2 antibody, or an isolated antigen-binding portion thereof, comprising:
(i) a heavy chain wherein the variable domain comprises H-CDR1 having an amino acid sequence as set forth in sequence SEQ ID NO: 11; H-CDR2 having an amino acid sequence as set forth in sequence SEQ ID NO: 12 and H-CDR3 having an amino acid sequence as set forth in sequence SEQ ID NO: 13; and
(ii) a light chain wherein the variable domain comprises L-CDR1 having an amino acid sequence as set forth in sequence SEQ ID NO: 16; L-CDR2 having an amino acid sequence as set forth in sequence SEQ ID NO: 17 and L-CDR3 having an amino acid sequence as set forth in sequence SEQ ID NO: 18.

The inventors have indeed generated a novel anti-IL-2 antibody which is able, when binding to IL-2, to *in vivo* induce Treg cells proliferation in an individual in need thereof. Surprisingly, as illustrated in the examples and contrary to the teachings of the prior art, the specific antibody of the invention in particular demonstrated both an *in vitro* ability to block binding of IL-2 to CD25 and an *in vivo* ability to stimulate Treg cells.

Even more surprisingly, the inventors demonstrate in the examples the ability of an antibody according to the invention, and in particular a complex of the invention comprising an antibody of the invention, or an antigen-binding portion thereof, and IL-2, to increase the Helios expression by Tregs, showing that the antibody, antigen-binding portion thereof, and complexes of the invention not only increase Treg numbers *in vivo,* but also boost their immunosuppressive functions.

The examples moreover show that the antibody, antigen-binding portion thereof, and complexes according to the invention are highly effective in mouse models of induced airway allergy and induced food allergy. In particular, administration of an active according to the invention led to a reduction in allergic markers, anatomical lesions and inflammation in both models, with physiological functions being ameliorated in treated mice.

The antibody, antigen-binding portion thereof, and complexes according to the invention moreover surprisingly demonstrated their ability to disseminate systemically throughout the body of the treated individual and to increase the half-life of IL-2 while still being well eliminated.

The examples moreover demonstrate their ability to be perfectly well tolerated by the organism of the treated individual, with the absence of any IL-2 mediated pulmonary edema following administration of high doses of a complex according to the invention. Contrary to the severe toxicities observed in the past following repeated administration of IL-2 to patients in order to counteract its short half-life, and in particular vascular leak syndrome (VLS), no VLS was observed following treatment with a complex according to the invention at any of the concentrations used.

**Item 2:** The antibody, or antigen-binding portion thereof, according to item 1, wherein:
- the variable domain of the heavy chain of the antibody has an amino acid sequence having at least 85% sequence identity with an amino acid sequence selected from the sequences set forth as sequence SEQ ID NO: 14 and SEQ ID NO: 15, and a biological activity of the same nature as the sequences set forth as sequence SEQ ID NO: 14 and SEQ ID NO: 15; and/or
- the variable domain of the light chain of the antibody an amino acid sequence having at least 85% sequence identity with an amino acid sequence selected from the sequences set forth as sequence SEQ ID NO: 19 and SEQ ID NO: 20, and a biological activity of the same nature as the sequences set forth as sequence SEQ ID NO: 19 and SEQ ID NO: 20.

**Item 3:** The antibody, or antigen-binding portion thereof, of item 1 or 2, said antibody being obtainable from the hybridoma deposited at the CNCM under reference CNCM 1-5726.

**Item 4:** The antibody, or antigen-binding portion thereof, according to any one of items 1 to 3, wherein the antibody is a chimeric or humanized antibody.

**Item 5:** The antibody, or antigen-binding portion thereof, according to any one of items 1 to 4, wherein the antigen-binding portion is selected from the group consisting of Fab, Fab', F(ab')₂, domain antibodies (dAbs, e.g., shark and camelid antibodies), portions including complementarity determining regions (CDRs), scFv, minibodies, diabodies, triabodies, and tetrabodies.

**Item 6:** The antibody, or antigen-binding portion thereof, according to any one of items 1 to 5, wherein the antibody, or antigen-binding portion thereof neutralizes *in vitro* the binding of human IL-2 to the trimeric receptor of IL-2 composed of CD25, CD122 and CD132.

**Item 7:** An IL-2 complex comprising the anti-human IL-2 antibody, or an antigen-binding portion thereof, according to any one of items 1 to 6, and a human IL-2, the antibody, or antigen-binding portion thereof, being non-covalently bound to the human IL-2.

**Item 8:** The anti-human IL-2 antibody, or antigen-binding portion thereof, as defined in any one of items 1 to 6, or the IL-2 complex according to item 7, wherein the antibody, antigen-binding portion thereof or IL-2 complex induces CD4(+) CD25(+) FoxP3(+) regulatory T cells proliferation *in vivo.*

**Item 9:** The anti-human IL-2 antibody, or antigen-binding portion thereof, as defined in any one of items 1 to 6 and 8, for its use in the prevention or treatment of respiratory and/or food allergies, or asthma, in a patient in need thereof.

**Item 10:** The anti-human IL-2 antibody, or antigen-binding portion thereof, for use according to item 9, wherein the antibody or antigen-binding portion thereof is administered to the patient in need thereof simultaneously, or sequentially, with human IL-2, in particular by the same route.

**Item 11:** The anti-human IL-2 antibody, or antigen-binding portion thereof, for use according to item 10, wherein the IL-2 complex according to item 8 is administered to the patient in need thereof.

**Item 12:** The anti-human IL-2 antibody, or antigen-binding portion thereof, for use according to any one of items 9 to 11, wherein the antibody, antigen-binding portion thereof or IL-2 complex is administered to the patient in need thereof simultaneously, or sequentially, with at least one food and/or respiratory allergen.

**Item 13:** An isolated nucleic acid encoding the anti-human IL-2 antibody, or an isolated antigen-binding portion thereof, as defined in any one of items 1 to 6, comprising in particular the nucleic acid sequences SEQ ID NO: 1, SEQ ID NO: 2, SEQ ID NO: 3, SEQ ID NO: 6, SEQ ID NO: 7 and SEQ ID NO: 8, and comprising more particularly the nucleic acid sequences (i) SEQ ID NO: 4 or SEQ ID NO: 5 and (ii) SEQ ID NO: 9 or SEQ ID NO: 10.

**Item 14:** An expression vector comprising the isolated nucleic acid of item 13 operably linked to a promoter.

**Item 15:** A kit for the treatment or prevention of respiratory and/or food allergies in a patient in need thereof, the kit comprising:
- at least one anti-human IL-2 antibody, or an antigen-binding portion thereof, as defined in any one of items 1 to 6; and
- at least one human IL-2.

### [DESCRIPTION OF THE FIGURES]

**Figure 1A** represents the ability of an antibody of the invention to bind to IL-2 measured through direct ELISA wherein IL-2 is added in wells coated with the said antibody. Variation of optical density (Ordinate) is observed and compared between (abscissa - from left to right) a control (CTL), a negative control addition of IL-2 in the absence of antibody coated in the wells (IL-2), and the actual test with IL-2 being added to the wells coated with the tested antibody.
**Figure 1B** represents assessment of the biological activity of the complex formed by an antibody of the invention and IL-2 (Ab/IL-2) by using CTLL-2 cell line which proliferates in presence of IL-2. The cells proliferation (Ordinate - %) is observed while the concentration of IL-2 is progressively increased (abscissa - Log [IL-2] pg/mL) ( 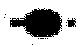 - IL-2) or with fixed IL-2 concentrations with increasing concentrations of an antibody of the invention ( 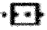 -IL-2 + Ab of the invention) or with increasing concentrations of a Ab/IL-2 complex of the invention ( 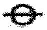 - Ab/IL-2 complex).
**Figure 2** represents measure of mouse primary CD4+ T cell proliferation (Ordinate - %) in the presence of increasing concentrations of IL-2 ( 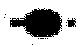 - IL-2) or of a complex according to the invention ( 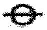 - Ab/IL-2 complex) (Abscissa - Log [Ab/IL-2] pg/mL).

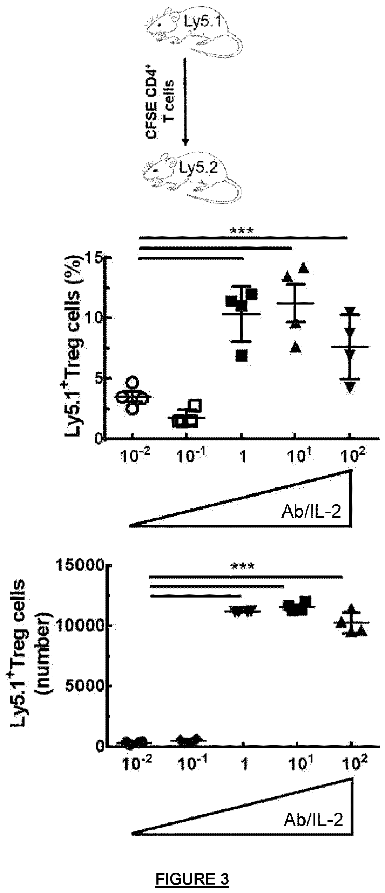

**Figure 3** represents the ability of a complex according to the invention (Ab/IL-2) to include T cell proliferation *in vivo* using adoptively transferred Ly5.1+ CD4+ T cells labeled with CSFE into Ly5.2 mice. Donor Ly5.1+ Treg cell frequencies (Top part - Ordinate (%)) and numbers (bottom part (Ordinate - (number)) are analyzed by flow cytometry in Ly5.2 recipient mice after treatment with a complex of the invention (Ab/IL-2) at different concentrations (abscissa) (1 corresponds to 1,5 µg of IL-2 + 15 µg of an antibody of the invention. 10⁻² corresponds to a concentration of 100 times less this concentration of both IL-2 and the antibody, 10⁻¹ corresponds to a concentration of 10 times less this concentration of both IL-2 and the antibody, 10¹ corresponds to 10 times this concentration of both IL-2 and the antibody, 10² corresponds to 100 times this concentration of both IL-2 and the antibody).
**Figure 4A** represents the ability of IL-2 or of a complex according to the invention to induce Treg cells in mice through the analyzes of splenic Tregs after IL-2 or complex treatment by flow cytometry.
   Treg cell frequencies (Top part - Ordinate (%)) and numbers (bottom part (Ordinate - (number)) are observed.
   Abscissa, from left to right: control (CTL), administration of only IL-2 (IL-2) and administration of a complex according to the invention (Ab/IL-2).
**Figure 4B** represents analyzed spleen naive (to part - Ordinate - Naive Treg MFI (A.U.)) or memory Treg (bottom part - Ordinate - Memory Treg MFI (A.U.)) subsets and the expression of ICOS, Helios and Neuropilin (NRP-1) on each subset by flow cytometry following treatment with PBS (Control - CTL), IL-2 alone (IL-2) or with a complex according to the invention (Ab/IL-2).
   Abscissa: for each marker observed (from left to right ICOS, NeuroP and Helios), three columns: from left to right CTL, IL-2 and then Ab/IL-2.
**Figure 5A** represents analysis of the biodistribution of a complex according to the invention after five daily injections to mice.
   Ordinate: Relative fluorescence intensity (IF/IFO) of the Ab/IL-2 complex.
   Abscissa: from left to right: Blood, Urine, Spleen, Lung.
**Figure 5B** represents measure of toxicological markers in blood with increasing concentrations of Ab/IL-2 complex.
   Ordinate: toxicological markers level intensity.
   Abscissa: From left to right: alanine transaminase (ALT), aspartate transaminase (AST), C reactive protein (CRP), creatinine (Crea). For each marker, the columns represent from left to right an increase of the complex concentration.
**Figure 5C** represents the evaluation of vascular leak syndrome (VLS) in mice treated with increasing concentrations of a Ab/IL-2 complex of the invention.
   Ordinate: Pulmonary weight loss (g).
   Abscissa: from left to right: Increasing concentrations of Ab/IL-2 complex.
**Figures 5D** **and** **5E** represents the determined clearance of the Ab/IL-2 complex obtained through measuring the blood (Figure 5D) and splenic (Figure 5E) concentrations of the complex at 0, 24 and 48 h after one injection.
   Ordinate: Optical Density (OD) of the Ab/IL-2 complex in blood.
   Abscissa: Time (h) after injection of the complex to the mice.
**Figure 6A** represents the protocol for obtaining a mouse model of HDM-induced airway inflammation.
**Figure 6B** represents observation of the variation of lung resistance in response to increasing concentrations of methacholine in control mice ( 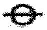 CTL), in the mice model of airway inflammation (asthmatic mice) ( 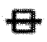 HDM) and in the mice model of airway inflammation (asthmatic mice) following administration of an Ab/IL-2 complex according to the invention ( 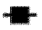 Ab/IL-2).
   Ordinate: Resistance (cH₂0.s/mL).
   Abscissa: Increasing concentrations of Methacholine (mg/mL).
**Figure 6C** represents the number of various inflammatory cells measured following bronchoalveolar lavage (BAL) from control mice ( 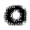 CTL), from the mice model of airway inflammation (asthmatic mice) ( 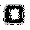 HDM) and from the mice model of airway inflammation (asthmatic mice) following administration of an Ab/IL-2 complex according to the invention (■ Ab/IL-2).
   Ordinate: cell number (*10³).
   Abscissa: from left to right: Eosinophil (Eo), Macrophage (Mo), polynuclear neutrophil (PNN).
**Figure 6D** represents the observation of pulmonary lesions in control mice ( 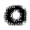 CTL), in the mice model of airway inflammation (asthmatic mice) ( 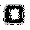 HDM) and in the mice model of airway inflammation (asthmatic mice) following administration of an Ab/IL-2 complex according to the invention (■ Ab/IL-2).
   Ordinate: Histological score.
   Abscissa: from left to right: Control (CTL), HDM, Ab/IL-2.
**Figure 6E** represents the observed effect of an Ab/IL-2 complex of the invention on circulating HDM-specific IgE as a reflection of the Th2 humoral response in control mice ( 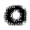 CTL), in the mice model of airway inflammation (asthmatic mice) ( 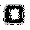 HDM) and in the mice model of airway inflammation (asthmatic mice) following administration of an Ab/IL-2 complex according to the invention (■ Ab/IL-2).
   Ordinate: Relative fluorescence intensity (IF/IFO) of specific IgE.
   Abscissa: from left to right: Control (CTL), HDM, Ab/IL-2.
**Figure 7A** represents the protocol for obtaining a mouse model of food allergy induced by intraperitoneal sensitization and oral challenges with wheat gliadins.
**Figure 7B** represents the measured effects of an Ab/IL-2 complex of the invention on body temperature after oral challenge of the model mice. Temperature (Ordinate (°C)) was measured in (abscissa, from left to right) control mice ( 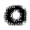 CTL), in the mice model of food allergy ( 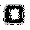 FA) and in the mice model of food allergy following administration of an Ab/IL-2 complex according to the invention (■ Ab/IL-2).
**Figure 7C** represents the implications (Ordinate : %) of the CD103+ dendritic cells subset (CD11c⁺ MHC11⁺ CD11b⁻ CD103⁺ DCs) in (abscissa, from left to right) control mice
   ( 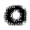 CTL), in the mice model of food allergy ( 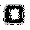 FA) and in the mice model of food allergy following administration of an Ab/IL-2 complex according to the invention (■ Ab/IL-2).
**Figure 7D** represents the observation of jejunum structural degradations in control mice ( 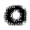 CTL), in the mice model of food allergy ( 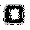 FA) and in the mice model of food allergy following administration of an Ab/IL-2 complex according to the invention (■ Ab/IL-2).
   Ordinate: Histological score.
   Abscissa: from left to right: Control (CTL), FA, Ab/IL-2.
**Figure 7E** represents the observed effect of an Ab/IL-2 complex of the invention on circulating wheat gliadin-specific IgE in control mice ( 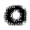 CTL), in the mice model of food allergy ( 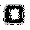 FA) and in the mice model of food allergy following administration of an Ab/IL-2 complex according to the invention (■ Ab/IL-2).
   Ordinate: Relative fluorescence intensity (IF/IFO) of specific IgE.
   Abscissa: from left to right: Control (CTL), HDM, Ab/IL-2.

### [DESCRIPTION OF THE SEQUENCES]

**SEQ ID NO: 1** represents the nucleic acid sequence coding for H-CDR1 of the anti-human IL-2 antibody of the invention:
   AGTAGTAGTTATTGCTGGCAC
**SEQ ID NO: 2** represents the nucleic acid sequence coding for H-CDR2 of the anti-human IL-2 antibody of the invention: 
CGCATATGTTATGAAGGTTCAATATACTATAGTCCATCCATCAAAAG
C
**SEQ ID NO: 3** represents the nucleic acid sequence coding for H-CDR3 of the anti-human IL-2 antibody of the invention:
   GAAAACTGGGGCTATGGACCCAACCCTTATGCTATGGACTAC
**SEQ ID NO:** 4 represents the nucleic acid sequence coding for the heavy variable domain of the anti-human IL-2 antibody of the invention, without a leader sequence: 
CAGATTCAGCTTAAGGAGTCTGGACCTGCTGTCATCAAGCCATCAC
AGTCACTGTCTCTCACCTGCATAGTCTCTGGATTCTCCATCACAAGTAGTAGTT
ATTGCTGGCACTGGATCCGCCAGCCCCCAGGAAAGGGGTTAGAGTGGATGGGG
CGCATATGTTATGAAGGTTCAATATACTATAGTCCATCCATCAAAAGCCGCAG
CACCATCTCCAGAGACACATCTCTGAACAAATTCTTTATCCAGCTGAGCTCTGT
GACAAATGAGGACACAGCCATGTACTACTGTTCCAGGGAAAACTGGGGCTATG
GACCCAACCCTTATGCTATGGACTACTGGGGTCAAGGAACCTCAGTCACCGTC
TCCTCA
**SEQ ID NO: 5** represents the nucleic acid sequence coding for the heavy variable domain of the anti-human IL-2 antibody of the invention, with a leader sequence: 
ATGAGACTACTAGGGTTTCTCCTGTGCTTGGCAGCAGCTCTAAAAA
GTGTTCTGTCCCAGATTCAGCTTAAGGAGTCTGGACCTGCTGTCATCAAGCCAT
CACAGTCACTGTCTCTCACCTGCATAGTCTCTGGATTCTCCATCACAAGTAGTA
GTTATTGCTGGCACTGGATCCGCCAGCCCCCAGGAAAGGGGTTAGAGTGGATG
GGGCGCATATGTTATGAAGGTTCAATATACTATAGTCCATCCATCAAAAGCCG
CAGCACCATCTCCAGAGACACATCTCTGAACAAATTCTTTATCCAGCTGAGCTC
TGTGACAAATGAGGACACAGCCATGTACTACTGTTCCAGGGAAAACTGGGGCT
ATGGACCCAACCCTTATGCTATGGACTACTGGGGTCAAGGAACCTCAGTCACC
GTCTCCTCA
**SEQ ID NO: 6** represents the nucleic acid sequence coding for L-CDR1 of the anti-human IL-2 antibody of the invention:
   CGCTCAAGTACTGGGGCTGTTACAACTAGTAACTATGCCAAC
**SEQ ID NO: 7** represents the nucleic acid sequence coding for L-CDR2 of the anti-human IL-2 antibody of the invention:
   GGTACCAGCAACCGAGCTCCA
**SEQ ID NO: 8** represents the nucleic acid sequence coding for L-CDR3 of the anti-human IL-2 antibody of the invention:
   GCTCTATGGTACAGCACCCATTTTGTT
**SEQ ID NO: 9** represents the nucleic acid sequence coding for the light variable domain of the anti-human IL-2 antibody of the invention, without a leader sequence: 
CAGGCTGTTGTGACTCAGGAATCTGCACTCACCACATCACCTGGTG
GAACAGTCATACTCACTTGTCGCTCAAGTACTGGGGCTGTTACAACTAGTAACT
ATGCCAACTGGGTCCAAGAAAAACCAGATCATTTATTCACTGGTCTAATAGGT
 
GGTACCAGCAACCGAGCTCCAGGTGTTCCTGTCAGATTCTCAGGCTCCCTGATT
GGAGACAAGGCTGCCCTCACCATCACAGGGGCACAGACTGAGGATGATGCAA
TGTATTTCTGTGCTCTATGGTACAGCACCCATTTTGTTTTCGGCGGTGGAACCA
AGGTCACTGTCCTA
**SEQ ID NO: 10** represents the nucleic acid sequence coding for the light variable domain of the anti-human IL-2 antibody of the invention, with a leader sequence: 
ATGGCCTGGACTTCACTTATACTCTCTCTCCTGGCTCTCTGCTCAGG
AGCCAGTTCCCAGGCTGTTGTGACTCAGGAATCTGCACTCACCACATCACCTG
GTGGAACAGTCATACTCACTTGTCGCTCAAGTACTGGGGCTGTTACAACTAGT
AACTATGCCAACTGGGTCCAAGAAAAACCAGATCATTTATTCACTGGTCTAAT
AGGTGGTACCAGCAACCGAGCTCCAGGTGTTCCTGTCAGATTCTCAGGCTCCCT
GATTGGAGACAAGGCTGCCCTCACCATCACAGGGGCACAGACTGAGGATGAT
GCAATGTATTTCTGTGCTCTATGGTACAGCACCCATTTTGTTTTCGGCGGTGGA
ACCAAGGTCACTGTCCTA
**SEQ ID NO: 11** represents the amino acid sequence of H-CDR1 of the anti-human IL-2 antibody of the invention:
   SSSYCWH
**SEQ ID NO: 12** represents the amino acid sequence of H-CDR2 of the anti-human IL-2 antibody of the invention:
   RICYEGSIYYSPSIKS
**SEQ ID NO: 13** represents the amino acid sequence of H-CDR3 of the anti-human IL-2 antibody of the invention:
   ENWGYGPNPYAMDY
**SEQ ID NO: 14** represents the amino acid sequence of the heavy variable domain of the anti-human IL-2 antibody of the invention, without a leader sequence: 
QIQLKESGPAVIKPSQSLSLTCIVSGFSITSSSYCWHWIRQPPGKGLEWM
GRICYEGSIYYSPSIKSRSTISRDTSLNKFFIQLSSVTNEDTAMYYCSRENWGYGPNP
YAMDYWGQGTSVTVSS
**SEQ ID NO: 15** represents the amino acid sequence of the heavy variable domain of the anti-human IL-2 antibody of the invention, with a leader sequence: 
MRLLGFLLCLAAALKSVLSQIQLKESGPAVIKPSQSLSLTCIVSGFSITSS
SYCWHWIRQPPGKGLEWMGRICYEGSIYYSPSIKSRSTISRDTSLNKFFIQLSSVTNE
DTAMYYCSRENWGYGPNPYAMDYWGQGTSVTVSS
**SEQ ID NO: 16** represents the amino acid sequence of L-CDR1 of the anti-human IL-2 antibody of the invention:
   RSSTGAVTTSNYAN
**SEQ ID NO: 17** represents the amino acid sequence of L-CDR2 of the anti-human IL-2 antibody of the invention:
   GTSNRAP
**SEQ ID NO: 18** represents the amino acid sequence of L-CDR3 of the anti-human IL-2 antibody of the invention:
   ALWYSTHFV
**SEQ ID NO: 19** represents the amino acid sequence of the light variable domain of the anti-human IL-2 antibody of the invention, without a leader sequence: 
QAVVTQESALTTSPGGTVILTCRSSTGAVTTSNYANWVQEKPDHLFTG
LIGGTSNRAPGVPVRFSGSLIGDKAALTITGAQTEDDAMYFCALWYSTHFVFGGGT
KVTVL
**SEQ ID NO: 20** represents the amino acid sequence of the light variable domain of the anti-human IL-2 antibody of the invention, with a leader sequence:

MAWTSLILSLLALCSGASSQAVVTQESALTTSPGGTVILTCRSSTGAVT
TSNYANWVQEKPDHLFTGLIGGTSNRAPGVPVRFSGSLIGDKAALTITGAQTEDDA
MYFCALWYSTHFVFGGGTKVTVL

### [DETAILED DESCRIPTION]

### Definitions

It must be noted that as used herein and in the appended claims, the singular forms **"a", "an",** and **"the"** include plural referents unless the context clearly dictates otherwise. Thus, for example, reference to "an antibody" includes a plurality of such antibodies, and so forth.

The terms **"about"** or **"approximately"** as used herein refer to the usual error range for the respective value readily known to the skilled person in this technical field. Reference to "about" a value or parameter herein includes (and describes) embodiments that are directed to that value or parameter per se. In some embodiments, the term "about" refers to ±10% of a given value. However, whenever the value in question refers to an indivisible object, such as a molecule or other object that would lose its identity once subdivided, then "about" refers to ± 1 of the indivisible object.

It is understood that aspects and embodiments of the present disclosure described herein include ***"having," "comprising," "consisting of,"*** and ***"consisting essentially of"*** aspects and **embodiments. The words *"have"* and** *"comprise,"* or variations such as *"has", "having", "comprises",* or *"comprising",* will be understood to imply the inclusion of the stated element(s) (such as a composition of matter or a method step) but not the exclusion of any other elements. The term *"consisting of"* implies the inclusion of the stated element(s), to the exclusion of any additional elements. The terms *"consisting essentially of"* implies the inclusion of the stated elements, and possibly other element(s) where the other element(s) do not materially affect the basic and novel characteristic(s) of the disclosure. It is understood that the different embodiments of the disclosure using the term *"comprising"* or equivalent cover the embodiments where this term is replaced with *"consisting of"* or *"consisting essentially of".*

Within the invention, the term **"allergy"** is synonymous with **"allergic response"** or **"allergic reaction".** Each of these terms refers to a state of immune responsiveness in an animal specific to an exogenous antigen (or **"allergen"**) that is not otherwise harmful to the animal.

As used herein, the phrases **"allergic response", "allergic reaction", "allergic symptom"** and the like, comprise one or more signs or symptoms selected from the group consisting of urticaria (e.g., hives), angioedema, rhinitis, asthma, vomiting, sneezing, runny nose, sinus inflammation, watery eyes, wheezing, bronchospasm, reduced peak expiratory flow (PEF), gastrointestinal distress, flushing, swollen lips, swollen tongue, reduced blood pressure, anaphylaxis, and organ dysfunction/failure. An "allergic response", "allergic reaction", "allergic symptom", etc., also includes immunological responses and reactions such as, e.g., increased IgE production and/or increased allergen-specific immunoglobulin production.

The term **"allergen,"** as used herein, includes any substance, chemical, particle or composition which is capable of stimulating an allergic response in a susceptible individual. Allergens may be contained within or derived from a food item such as, e.g., dairy products (e.g., cow's milk), egg, celery, sesame, wheat, soy, fish, shellfish, sugars (e.g., sugars present on meat such as alpha-galactose), peanuts, other legumes (e.g., beans, peas, soybeans, etc.), and tree nuts. Alternatively, an allergen may be contained within or derived from a non-food item such as, e.g., dust (e.g., containing dust mite), pollen, insect venom (e.g., venom of bees, wasps, mosquitos, fire ants, etc.), mold, animal fur, animal dander, wool, latex, metals (e.g., nickel), household cleaners, detergents, medication, cosmetics (e.g., perfumes, etc.), drugs (e.g., penicillin, sulfonamides, salicylate, etc.), therapeutic monoclonal antibodies (e.g., cetuximab), ragweed, grass and birch. Exemplary pollen allergens include, e.g., tree pollens such as birch pollen, cedar pollen, oak pollen, alder pollen, hornbeam pollen, aesculus pollen, willow pollen, poplar pollen, plantanus pollen, tilia pollen, olea pollen, Ashe juniper pollen, and Alstonia scholaris pollen.

A **"symptom"** of an allergic response refers to any measure of the aforesaid immune responsiveness, for example on the molecular level, the cellular level, organ level, systemic level, and/or organism level, as for example illustrated in the examples. Symptoms may include generalized phenomena such as inflammation, respiratory complaints, swelling, or distress typically associated with allergy, rhinitis, edema, and allergic skin disorders including but not limited to atopic dermatitis (for example eczema), urticaria and angioedema, and allergic contact dermatitis.

More specific phenomena that are "symptoms" of an allergic response include any measurable or observable change, for example at the cellular level, including but not limited to local or systemic changes in cell populations, eosinophilia, recruitment and/or activation of immune cells, including, for example, mast cells and/or basophils, changes in antigen-presenting cells (including but not limited to FcεRI-bearing dendritic cells), intracellular or molecular changes, including measurement or observations of one or more steps in an immunological cascade, release of intracellular compounds that mediate an allergic response (e.g., mediators), and changes in one or more cytokines (e.g., IL-3, IL-5, IL-9, IL-4, or IL-13) or related compounds or antagonists thereof. The skilled person will understand that certain symptoms as defined herein are more readily measured than others, and some are measured through subjective assessment or self-assessment of the symptom. For other symptoms, there are convenient or rapid assays or measurements well known in the art for objectively assessing changes.

Within the invention, **"anti-human IL-2 antibody"** and **"anti-human IL-2 antigen-binding portion thereof"** intend to refer to immunoglobulins, or antigen-binding portion thereof, able to specifically recognize and bind to human Interleukin 2 (hIL-2). Depending on the antibody amino acid sequence of the constant region of its heavy chains, immunoglobulins can be assigned to different classes. There are five major classes (i.e., isotypes) of immunoglobulins: IgA, IgD, IgE, IgG, and IgM, and several of these may be further divided into subclasses (subtypes), e.g., IgG-i , lgG₂, lgG3, lgG₄, IgA₁ and lgA₂. The heavy-chain constant regions that correspond to the different classes of immunoglobulins are called alpha, delta, epsilon, gamma, and mu, respectively. The subunit structures and three-dimensional configurations of different classes of immunoglobulins are well known.

An **"antigen-binding portion"** of an antibody according to the invention intends to refer to any antigen-binding portion that competes with the intact antibody for specific binding to the targeted human IL-2, fusion proteins comprising an antigen-binding portion, and any other modified configuration of the immunoglobulin molecule that comprises an antigen recognition site. Antigen-binding portions include, for example, Fab, Fab', F(ab')₂, domain antibodies (dAbs, e.g., shark and camelid antibodies), portions including complementarity determining regions (CDRs), single chain variable fragment antibodies (scFv), minibodies, diabodies, triabodies, and tetrabodies.

A **"variable domain",** or **"variable region",** of an antibody refers to the variable domain of the antibody light chain (VL) or the variable domain of the antibody heavy chain (VH), either alone or in combination. As known in the art, the variable domains of the heavy and light chains each consist of four framework regions (FRs) connected by three complementarity determining regions (CDRs) also known as hypervariable regions, and contribute to the formation of the antigen-binding site of antibodies. If variants of a subject variable domain are desired, particularly with substitution in amino acid residues outside a CDR (i.e., in the framework region), appropriate amino acid substitution, in particular conservative amino acid substitution, can be performed.

As known in the art, a **"constant region"** of an antibody refers to the constant region of the antibody light chain or the constant region of the antibody heavy chain, either alone or in combination.

As used herein, **"monoclonal antibody"** refers to an antibody obtained from a population of substantially homogeneous antibodies, i.e., the individual antibodies comprising the population are identical except for possible naturally-occurring mutations that may be present in minor amounts. Monoclonal antibodies are highly specific, being directed against a single antigenic site. Furthermore, in contrast to polyclonal antibody preparations, which typically include different antibodies directed against different determinants (epitopes), each monoclonal antibody is directed against a single determinant on the antigen. The modifier **"monoclonal"** indicates the character of the antibody as being obtained from a substantially homogeneous population of antibodies, and is not to be construed as requiring production of the antibody by any particular method.

As used herein, **"humanized"** antibody refers to forms of non-human (e.g., murine) antibodies that are chimeric immunoglobulins, immunoglobulin chains, or portions thereof (such as Fv, Fab, Fab', F(ab')2 or other antigen-binding subsequences of antibodies) that contain minimal sequence derived from non-human immunoglobulin. In some embodiments, humanized antibodies are human immunoglobulins (recipient antibody) in which residues from a CDR of the recipient are replaced by residues from a CDR of a non-human species (donor antibody) such as mouse, rat, or rabbit having the desired specificity, affinity, and capacity. The humanized antibody may comprise residues that are found neither in the recipient antibody nor in the imported CDR or framework sequences, but are included to further refine and optimize antibody performance.

A **"human"** antibody is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen binding residues.

The term **"chimeric antibody"** refers to an antibody which comprises the variable domain of the heavy chain and the variable domain of the light chain of a non-human antibody according to the invention, and the heavy and light constant domains of a human antibody.

According to the invention, the term **"humanized antibody"** refers to an antibody having variable region framework and constant regions from a human antibody but retains the CDRs a non-human antibody of the invention.

It is understood in the art that variable regions of an antibody, or an antigen-binding portion thereof, possesses a variable light chain (VL) and a variable heavy chain (VH). A **"complementary determining regions"** (CDR) of a light or heavy chain is conventionally referred to under the terms, respectively, "L-CDR" or "H-CDR".

The term **"Fab"** denotes an antibody fragment having a molecular weight of about 50,000 and antigen binding activity, in which about a half of the N-terminal side of heavy chain and the entire light chain, among fragments obtained by treating IgG with a protease, papaine, are bound together through a disulfide bond.

The term **"F(ab')2"** refers to an antibody fragment having a molecular weight of about 100,000 and antigen binding activity, which is slightly larger than the Fab bound via a disulfide bond of the hinge region, among fragments obtained by treating IgG with a protease, pepsin.

The term "**Fab'**" refers to an antibody fragment having a molecular weight of about 50,000 and antigen binding activity, which is obtained by cutting a disulfide bond of the hinge region of the F(ab')2.

A single chain Fv ("**scFv**") polypeptide is a covalently linked heavy-chain variable domain:light-chain variable domain heterodimer which is usually expressed from a gene fusion including heavy-chain variable domain and light-chain variable domain encoding genes linked by a peptide-encoding linker.

The term **"diabodies"** refers to small antibody fragments with two antigen-binding sites, which fragments comprise a heavy-chain variable domain connected to a light-chain variable domain in the same polypeptide chain (VH-VL). By using a linker that is too short to allow pairing between the two domains on the same chain, the domains are forced to pair with the complementary domains of another chain and create two antigen-binding sites.

**"Minibodies"** are a form of antibody that has a lower molecular weight than a full-length antibody while maintaining the bivalent binding properties for the antibody. The minibody format is a homodimer with each monomer having a single-chain variable fragment (scFv) linked to the human IgG1 CH3 domain.

The term **"triabodies"** refers to trivalent antibodies comprising three peptide chains, each of which contains one heavy-chain variable domain and one light-chain variable domain joined by a linker that is exceedingly short (e.g., a linker composed of 1-2 amino acids) to permit intramolecular association of heavy-chain variable domain and light-chain variable domain domains within the same peptide chain. In order to fold into their native structure, peptides configured in this way typically trimerize so as to position the heavy-chain variable domain and light-chain variable domain of neighbouring peptide chains spatially proximal to one another to permit proper folding (see Holliger et al., Proc. Natl. Acad. Sci. USA 90:6444-48, 1993).

As used herein, the term **"tetrabodies"** refers to a complex including four antigen-binding domains, where the four antigen-binding domains may be directed towards the same or different epitopes. Tetrabodies are constructed with the amino acid terminus of a light-chain variable domain or heavy-chain variable domain domain, i.e., without any linker sequence. A tetrabody can be combination of three single chain antibodies.

As known in the art, **"polynucleotide"** or **"nucleic acid",** as used interchangeably herein, refer to chains of nucleotides of any length, and include DNA and RNA. The nucleotides can be deoxyribonucleotides, ribonucleotides, modified nucleotides or bases, and/or their analogs, or any substrate that can be incorporated into a chain by DNA or RNA polymerase. A polynucleotide may comprise modified nucleotides, such as methylated nucleotides and their analogs. If present, modification to the nucleotide structure may be imparted before or after assembly of the chain. The sequence of nucleotides may be interrupted by non-nucleotide components. A polynucleotide may be further modified after polymerization, such as by conjugation with a labeling component.

An **"IL-2 complex"** within the meaning of the invention, refers to a complex comprising at least one antibody, or antigen-binding portion thereof, of the present invention that specifically binds IL-2 and at least one IL-2 cytokine molecule. The complex comprises an antibody and an IL-2 molecule that are associated by non-covalent force.

As used herein, the term **"polypeptide"** refers to a molecule comprising amino acid residues linked by peptide bonds. The amino acids are identified by either the single-letter or three-letter designations. The term "protein" as used herein is synonymous with the term "polypeptide" and may also refer to two or more polypeptides. Thus, the terms "protein", "peptide" and "polypeptide" can be used interchangeably. Polypeptides may optionally be modified (e.g., glycosylated, phosphorylated, acylated, farnesylated, prenylated, sulfonated, and the like) to add functionality. It will be understood that, as a result of the degeneracy of the genetic code, a multitude of nucleotide sequences encoding a given polypeptide may be produced.

By **"isolated"** it is meant, when referring to an antibody according to the invention or to a nucleotide sequence, that the indicated molecule is present in the substantial absence of other biological macromolecules of the same type. The term "isolated" as used herein preferably means at least 75% by weight, more preferably at least 85% by weight, more preferably still at least 95% by weight, and most preferably at least 98% by weight, of biological macromolecules of the same type are present.

Within the meaning of the invention, the terms **"patient", "subject" "recipient"** or **"individual"** are used interchangeably and intends to refer to a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In some exemplary embodiments, the individual or recipient is a human. Those terms intend to refer to individual in need of receiving or having received a kidney transplantation.

As used herein, the terms **"in need thereof"** relating to a patient means any subject who: (a) is prone to allergic reactions or responses when exposed to one or more allergens; (b) has previously exhibited an allergic response or reaction to one or more allergens; (c) has a known history of allergies; and/or (d) exhibits a sign or symptom of an allergic response or anaphylaxis.

The terms **"vector"** and **"expression vector"** mean the vehicle by which a DNA or RNA sequence (e.g. a foreign gene) can be introduced into a host cell, so as to transform the host and promote expression (e.g. transcription and translation) of the introduced sequence.

A **"host cell"** within the meaning of the invention includes an individual cell or cell culture that can be or has been a recipient for vector(s) for incorporation of polynucleotide inserts. Host cells include progeny of a single host cell, and the progeny may not necessarily be completely identical (in morphology or in genomic DNA complement) to the original parent cell due to natural, accidental, or deliberate mutation. A host cell includes cells transfected and/or transformed in vivo with a polynucleotide of this disclosure.

Within the meaning of the invention, the expression **"increase in the expression of"** intends to mean that the expression of the concerned increased item is above the normal or reference expression of the concerned item. The increase of expression may be partial or total.

In the invention, the expression of a Helios at a cell surface of Treg cells is increased, as disclosed in the Examples, following administration to an individual of an antibody of the invention, in particular of an antibody of the invention in a form of a complex with IL-2.

As used herein, the term **"administration simultaneously"** refers to administration of 2 active ingredients by the same route and at the same time or at substantially the same time. The term **"administration separately"** refers to an administration of 2 active ingredients at the same time or at substantially the same time by different routes. The term **"administration sequentially"** refers to an administration of 2 active ingredients at different times, the administration route being identical or different.

Within the disclosure, the term **"significantly"** used with respect to change intends to mean that the observe change is noticeable and/or it has a statistic meaning.

In the context of the present invention, the terms **"prevent",** or **"preventing"** denotes the reduction to a lesser degree of the risk or of the probability of occurrence of a given phenomenon, that is to say, in the present invention, allergies, in particular respiratory and food allergies, as well as asthma.

In the particular case of allergies, these terms moreover include allergy desensitization therapy, the preventive treatment resulting in a reduction in the symptoms of allergy (e.g. , reduction in rhinitis, allergic conjunctivitis, circulating levels of IgE, and/or circulating levels of histamine) or even can lead to a state of non-allergic reactivity.

As used herein, the terms **"treating"** or **"treat"** include alleviation of the symptoms associated with a specific disorder or condition and/or elimination of said symptoms, in particular while said symptoms are affecting an individual.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention. All publications mentioned herein are incorporated herein by reference to disclose and describe the methods and/or materials in connection with which the publications are cited.

The list of sources, ingredients, and components as described hereinafter are listed such that combinations and mixtures thereof are also contemplated and within the scope herein.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

All lists of items, such as, for example, lists of ingredients, are intended to and should be interpreted as Markush groups. Thus, all lists can be read and interpreted as items "selected from the group consisting of the list of items and combinations and mixtures thereof."

### Antibody and antigen-binding portion thereof of the invention

As previously indicated, a first object of the invention relates to an isolated anti-human IL-2 antibody, or an isolated antigen-binding portion thereof.

An anti-human IL-2 antibody, or an isolated antigen-binding portion thereof, of the invention comprises a heavy chain wherein the variable domain comprises H-CDR1 having an amino acid sequence as set forth in sequence SEQ ID NO: 11; H-CDR2 having an amino acid sequence as set forth in sequence SEQ ID NO: 12 and H-CDR3 having an amino acid sequence as set forth in sequence SEQ ID NO: 13.

An anti-human IL-2 antibody, or an isolated antigen-binding portion thereof, of the invention further comprises a light chain wherein the variable domain comprises L-CDR1 having an amino acid sequence as set forth in sequence SEQ ID NO: 16; L-CDR2 having an amino acid sequence as set forth in sequence SEQ ID NO: 17 and L-CDR3 having an amino acid sequence as set forth in sequence SEQ ID NO: 18.

An antibody of the invention is in particular a monoclonal antibody.

An antibody of the invention may be a chimeric or humanized antibody.

An antibody of the invention may in particular be such that:
- the variable domain of the heavy chain of the antibody has an amino acid sequence having at least 85% sequence identity with an amino acid sequence selected from the sequences set forth as sequence SEQ ID NO: 14 and SEQ ID NO: 15, and a biological activity of the same nature as the sequences set forth as sequence SEQ ID NO: 14 and SEQ ID NO: 15; and/or
- the variable domain of the light chain of the antibody an amino acid sequence having at least 85% sequence identity with an amino acid sequence selected from the sequences set forth as sequence SEQ ID NO: 19 and SEQ ID NO: 20, and a biological activity of the same nature as the sequences set forth as sequence SEQ ID NO: 19 and SEQ ID NO: 20.

In a variable domain of the heavy chain of an antibody of the invention having an amino acid sequence having at least 85% sequence identity with an amino acid sequence selected from the sequences set forth as sequence SEQ ID NO: 14 and SEQ ID NO: 15, the H-CDR1 has 100% sequence identity with the amino acid sequence set forth as sequence SEQ ID NO: 11; the H-CDR2 has 100% sequence identity with the amino acid sequence set forth as sequence SEQ ID NO: 12 and the H-CDR3 has 100% sequence identity with the amino acid sequence set forth as sequence SEQ ID NO: 13.

Similarly, in a variable domain of the light chain of an antibody of the invention having an amino acid sequence having at least 85% sequence identity with an amino acid sequence selected from the sequences set forth as sequence SEQ ID NO: 19 and SEQ ID NO: 20, the L-CDR1 has 100% sequence identity with the amino acid sequence set forth as sequence SEQ ID NO: 16; the L-CDR2 has 100% sequence identity with the amino acid sequence set forth as sequence SEQ ID NO: 17 and the L-CDR3 has 100% sequence identity with the amino acid sequence set forth as sequence SEQ ID NO: 18.

A biological activity of the same nature regarding these sequences is the capacity to bind to IL-2, in particular to human IL-2 (hIL-2), more particularly to bind to the same epitope of IL-2, in particular of hIL-2, as the sequences of reference indicated above.

Said antibodies may be assayed for specific binding by any method known in the art. Many different competitive binding assay format(s) can be used for epitope binding. The immunoassays which can be used include, but are not limited to, competitive assay systems using techniques such as western blots, radioimmunoassays, ELISA, "sandwich" immunoassays, immunoprecipitation assays, precipitin assays, gel diffusion precipitin assays, immunoradiometric assays, fluorescent immunoassays, protein A immunoassays, and complement-fixation assays. Such assays are routine and well known in the art (see, e.g., Ausubel et al., eds, 1994 Current Protocols in Molecular Biology, Vol. 1, John Wiley & sons, Inc., New York). For example, the BIACORE^{®} (GE Healthcare, Piscaataway, NJ) is one of a variety of surface plasmon resonance assay format that are routinely used to epitope bin panels of monoclonal antibodies. Additionally, routine cross-blocking assays such as those described in Antibodies, A Laboratory Manual, Cold Spring Harbor Laboratory, Ed Harlow and David Lane, 1988, can be performed.

As described herein, an amino acid sequence having at least 85% sequence identity with a reference amino acid sequence encompasses amino acid sequences having at least 86%, 87%, 88%, 89%, 90%, 91%, 92%, 93%, 94%, 95%, 96%, 97%, 98% and 99% sequence identity with the said reference amino acid sequence, and also a biological activity of the same nature as the said reference amino acid sequence.

The terms **"sequence homology"** or **"sequence identity"** or "homology" or **"identity"** are used interchangeably herein. For the purpose of the invention, it is defined here that in order to determine the percentage of sequence homology or sequence identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region.

A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences or between two nucleotide sequences may be determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE.

For the purpose of the invention, the NEEDLE program from the EMBOSS package was used (version 2.8.0 or higher, EMBOSS: The European Molecular Biology Open Software Suite (2000) Rice, P. LongdenJ. and Bleasby,A. Trends in Genetics 16, (6) pp276- 277, http://emboss.bioinformatics.nl/). For protein sequences EBLOSUM62 is used for the substitution matrix. For nucleotide sequence, EDNAFULL is used. The optional parameters used are a gap opening penalty of 10 and a gap extension penalty of 0.5. No end gap penalty is added. In the Output section, Yes has been indicated in response to the question "Brief identity and similarity" and "SRS pairwise" indicated as Output alignment format.

After alignment by the program NEEDLE as described above the percentage of sequence identity between a query sequence and a sequence of the invention is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labeled in the output of the program as "longest-identity".

The similarity of nucleotide and amino acid sequences, i.e. the percentage of sequence identity, can be determined via sequence alignments using several other art-known algorithms, preferably with the mathematical algorithm of Karlin and Altschul (Karlin & Altschul (1993) Proc. Natl. Acad. Sci. USA 90: 5873-5877), with hmmalign (HMMER package, http://hmmer.wustl.edu/) or with the CLUSTAL algorithm (Thompson, J. D., Higgins, D. G. & Gibson, T. J. (1994) Nucleic Acids Res. 22, 4673-80) available e.g. on https://www.ebi.ac.uk/Tools/msa/clustalo/ or the GAP program (mathematical algorithm of the University of Iowa) or the mathematical algorithm of Myers and Miller (1989 - Cabios 4: 11-17) or Clone Manager 9. Preferred parameters used are the default parameters as they are set on https://www.ebi.ac.uk/Tools/msa/clustalo/.

The grade of sequence identity (sequence matching) may be calculated using e.g. BLAST, BLAT or BlastZ (or BlastX). A similar algorithm is incorporated into the BLASTN and BLASTP programs of Altschul et al (1990) J. Mol. Biol. 215, 403-410. BLAST polynucleotide searches are performed with the BLASTN program, score = 100, word length = 12, to obtain polynucleotide sequences that are homologous to those nucleic acids which encode the relevant protein.

BLAST protein searches are performed with the BLASTP program, score = 50, word length = 3, to obtain amino acid sequences homologous to the SHC polypeptide. To obtain gapped alignments for comparative purposes, Gapped BLAST is utilized as described in Altschul et al (1997) Nucleic Acids Res. 25, 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1: 154-162) or Markov random fields. When percentages of sequence identity are referred to in the present application, these percentages are calculated in relation to the full length of the longer sequence, if not specifically indicated otherwise.

In particular embodiments, % identity between two sequences is determined using CLUSTAL O (version 1.2.4).

An antibody of the invention can in particular be obtainable from the hybridoma deposited at the CNCM under reference CNCM 1-5726 on July 30, 2021.

The present invention further relates to an isolated nucleic acid encoding an anti-human IL-2 antibody, or an isolated antigen-binding portion thereof, according to the invention.

A nucleic acid sequence according to the invention in particular comprises:
- a nucleic acid sequence having at least 85% sequence identity with the nucleic acid sequence set forth as sequence SEQ ID NO: 1, and a biological activity of the same nature (i.e. the ability to code for H-CDR1 as defined above of sequence SEQ ID NO: 11);
- a nucleic acid sequence having at least 85% sequence identity with the nucleic acid sequence set forth as sequence SEQ ID NO: 2, and a biological activity of the same nature (i.e. the ability to code for H-CDR2 as defined above of sequence SEQ ID NO: 12);
- a nucleic acid sequence having at least 85% sequence identity with the nucleic acid sequence set forth as sequence SEQ ID NO: 3, and a biological activity of the same nature (i.e. the ability to code for H-CDR3 as defined above of sequence SEQ ID NO: 13);
- a nucleic acid sequence having at least 85% sequence identity with the nucleic acid sequence set forth as sequence SEQ ID NO: 6, and a biological activity of the same nature (i.e. the ability to code for L-CDR1 as defined above of sequence SEQ ID NO: 16);
- a nucleic acid sequence having at least 85% sequence identity with the nucleic acid sequence set forth as sequence SEQ ID NO: 7, and a biological activity of the same nature (i.e. the ability to code for L-CDR2 as defined above of sequence SEQ ID NO: 17); and
- a nucleic acid sequence having at least 85% sequence identity with the nucleic acid sequence set forth as sequence SEQ ID NO: 8, and a biological activity of the same nature (i.e. the ability to code for L-CDR3 as defined above of sequence SEQ ID NO: 18).

A nucleic acid sequence according to the invention may in particular comprise the nucleic acid sequences SEQ ID NO: 1, SEQ ID NO: 2, SEQ ID NO: 3, SEQ ID NO: 6, SEQ ID NO: 7 and SEQ ID NO: 8.

In particular, a nucleic acid sequence according to the invention may comprise:
- a nucleic acid sequence having at least 85% sequence identity with the nucleic acid sequence set forth as sequence SEQ ID NO: 4 or with the nucleic acid sequence set forth as sequence SEQ ID NO: 5, and a biological activity of the same nature (i.e. the ability to code for variable domain of the heavy chain (HV) as defined above, respectively, of sequence SEQ ID NO: 14 (without a leader sequence) or of sequence SEQ ID NO: 15 (with a leader sequence)); and
- a nucleic acid sequence having at least 85% sequence identity with the nucleic acid sequence set forth as sequence SEQ ID NO: 9 or with the nucleic acid sequence set forth as sequence SEQ ID NO: 10, and a biological activity of the same nature (i.e. the ability to code for the variable domain of the light chain (HV) as defined above, respectively, of sequence SEQ ID NO: 19 (without a leader sequence) or of sequence SEQ ID NO: 20 (with a leader sequence)).

As described herein, a nucleic acid sequence having at least 85% sequence identity with a reference nucleic acid sequence encompasses nucleic acid sequences having at least 86%, 87%, 88%, 89%, 90%, 91%, 92%, 93%, 94%, 95%, 96%, 97%, 98% and 99% sequence identity with the said reference nucleic acid sequence, and also a biological activity of the same nature as the said reference nucleic acid sequence.

A nucleic acid sequence according to the invention may in particular comprise the nucleic acid sequences:
(i) SEQ ID NO: 4 or SEQ ID NO: 5; and
(ii) SEQ ID NO: 9 or SEQ ID NO: 10.

Modifications and changes may be made in the structure of the antibodies of the present invention, and in the DNA sequences encoding them, and still obtain a functional molecule that encodes an antibody with desirable characteristics.

In making the changes in the amino sequences, the hydropathic index of amino acids may be considered. The importance of the hydropathic amino acid index in conferring interactive biologic function on a protein is generally understood in the art. It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of their hydrophobicity and charge characteristics these are: isoleucine (+4.5); valine (+4.2); leucine (+3.8) ; phenylalanine (+2.8); cysteine/cystine (+2.5); methionine (+1.9); alanine (+1.8); glycine (-0.4); threonine (-0.7); serine (-0.8); tryptophane (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

Engineered antibodies of the invention include those in which modifications have been made to framework residues within VH and/or VL, e.g. to improve the properties of the antibody. Typically such framework modifications are made to decrease the immunogenicity of the antibody. For example, one approach is to "backmutate" one or more framework residues to the corresponding germline sequence. More specifically, an antibody that has undergone somatic mutation may contain framework residues that differ from the germline sequence from which the antibody is derived. Such residues can be identified by comparing the antibody framework sequences to the germline sequences from which the antibody is derived. To return the framework region sequences to their germline configuration, the somatic mutations can be "backmutated" to the germline sequence by, for example, site-directed mutagenesis or PCR-mediated mutagenesis. Such "backmutated" antibodies are also intended to be encompassed by the invention.

Another type of framework modification involves mutating one or more residues within the framework region, or even within one or more CDR regions, to remove T cell - epitopes to thereby reduce the potential immunogenicity of the antibody. This approach is also referred to as "deimmunization" and is described in further detail in US20030153043.

In addition or alternative to modifications made within the framework or CDR regions, antibodies of the invention may be engineered to include modifications within the Fc region, typically to alter one or more functional properties of the antibody, such as serum half-life, complement fixation, Fc receptor binding, and/or antigen-dependent cellular cytotoxicity. Furthermore, an antibody of the invention may be chemically modified (e.g., one or more chemical moieties can be attached to the antibody) or be modified to alter its glycosylation, again to alter one or more functional properties of the antibody. Each of these embodiments is described in further detail below. The numbering of residues in the Fc region is that of the EU index of Kabat.

In one embodiment, the hinge region of CH1 is modified such that the number of cysteine residues in the hinge region is altered, e.g., increased or decreased. This approach is described further in US5,677,425. The number of cysteine residues in the hinge region of CH1 is altered to, for example, facilitate assembly of the light and heavy chains or to increase or decrease the stability of the antibody.

In another embodiment, the Fc hinge region of an antibody is mutated to decrease the biological half-life of the antibody. More specifically, one or more amino acid mutations are introduced into the CH2-CH3 domain interface region of the Fc-hinge fragment such that the antibody has impaired Staphylococcyl protein A (SpA) binding relative to native Fc-hinge domain SpA binding. This approach is described in further detail in US6,165,745.

In another embodiment, the antibody is modified to increase its biological half-life. Various approaches are possible. For example, one or more of the following mutations can be introduced: T252L, T254S, T256F, as described in US6,277,375. Alternatively, the antibody can be altered within the CH1 or the constant region of the light chain (CL) to contain a salvage receptor binding epitope taken from two loops of a CH2 domain of an Fc region of an IgG, as described in US5,869,046 and US6,121,022.

A further object of the present invention relates to an expression vector comprising an isolated nucleic acid sequence according to the invention operably linked to a promoter.

Such vectors may comprise regulatory elements, such as a promoter, enhancer, terminator and the like, to cause or direct expression of said antibody upon administration to a subject. Examples of promoters and enhancers used in the expression vector for animal cell include early promoter and enhancer of SV40 (Mizukami T. et al. 1987), LTR promoter and enhancer of Moloney mouse leukemia virus (Kuwana Y et al. 1987), promoter (Mason JO et al. 1985) and enhancer (Gillies SD et al. 1983) of immunoglobulin H chain and the like.

Any expression vector for animal cell can be used, so long as a gene encoding the human antibody C region can be inserted and expressed. Examples of suitable vectors include pAGE107 (Miyaji H et al. 1990), pAGE103 (Mizukami T et al. 1987), pHSG274 (Brady G et al. 1984), pKCR (O'Hare K et al. 1981), pSG1 beta d2-4-(Miyaji H et al. 1990) and the like. Other examples of plasmids include replicating plasmids comprising an origin of replication, or integrative plasmids, such as for instance pUC, pcDNA, pBR, and the like. Other examples of viral vector include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO 94/19478.

A further object of the present invention relates to a host cell which has been transfected, infected or transformed by a nucleic acid and/or a expression vector according to the invention.

The term "transformation" means the introduction of a "foreign" (i.e. extrinsic or extracellular) gene, DNA or RNA sequence to a host cell, so that the host cell will express the introduced gene or sequence to produce a desired substance, typically a protein or enzyme coded by the introduced gene or sequence. A host cell that receives and expresses introduced DNA or RNA bas been "transformed".

The nucleic acids of the invention may be used to produce an antibody of the invention, or an antigen-binding portion thereof, in a suitable expression system. The term "expression system" means a host cell and compatible vector under suitable conditions, e.g. for the expression of a protein coded for by foreign DNA carried by the vector and introduced to the host cell.

Common expression systems include E. coli host cells and plasmid vectors, insect host cells and Baculovirus vectors, and mammalian host cells and vectors. Other examples of host cells include, without limitation, prokaryotic cells (such as bacteria) and eukaryotic cells (such as yeast cells, mammalian cells, insect cells, plant cells, etc.). Specific examples include E.coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from lymphoblasts, fibroblasts, embryonic cells, epithelial cells, nervous cells, adipocytes, etc.). Examples also include mouse SP2/0-Ag14 cell (ATCC CRL1581), mouse P3X63-Ag8.653 cell (ATCC CRL1580), CHO cell in which a dihydrofolate reductase gene (hereinafter referred to as "DHFR gene") is defective (Urlaub G et al; 1980), rat YB2/3HL.P2.G11.16Ag.20 cell (ATCC CRL1662, hereinafter referred to as "YB2/0 cell"), and the like.

The present invention also relates to a method of producing a recombinant host cell expressing an antibody, or an antigen-binding portion thereof, according to the invention, said method comprising the steps of: (i) introducing *in vitro* or *ex vivo* a nucleic acid or an expression vector as described above into a competent host cell, (ii) culturing *in vitro* or *ex vivo* the recombinant host cell obtained and (iii), optionally, selecting the cells which express and/or secrete said antibody. Such recombinant host cells can be used for the production of antibodies, or an antigen-binding portion thereof, of the invention.

In another particular embodiment, the method comprises the steps of:
(i) culturing the hybridoma CNCM 1-5726 under conditions suitable to allow expression of 16D3-C1 antibody; and
(ii) recovering the expressed antibody.

Antibodies of the invention are suitably separated from the culture medium by conventional immunoglobulin purification procedures such as, for example, protein A-Sepharose, hydroxylapatite chromatography, gel electrophoresis, dialysis, or affinity chromatography.

As demonstrated in the examples, an antibody, or antigen-binding portion thereof, according to the invention in particular surprisingly neutralizes *in vitro* the binding of human IL-2 to the trimeric receptor of IL-2 composed of CD25, CD122 and CD132.

Moreover, an antibody, or antigen-binding portion thereof, according to the invention is in particular able to induce *in vivo* proliferation of CD4(+) CD25(+) FoxP3(+) regulatory T cells.

### Complex according to the invention

As previously indicated, a further object of the invention relates to an IL-2 complex comprising:
- an anti-IL-2 antibody, or an antigen-binding portion thereof, according to the invention, as defined above, in particular an anti-human IL-2 antibody, or an antigen-binding portion thereof, according to the invention, as defined above; and
- an IL-2, in particular a human IL-2,
the antibody, or antigen-binding portion thereof, being non-covalently bound to the human IL-2.

As previously mentioned, a complex according to the invention is in particular able to induce *in vivo* proliferation of CD4(+) CD25(+) FoxP3(+) regulatory T cells.

As developed here-after, a complex according to the invention can be used in the prevention or treatment of respiratory and/or food allergies, or asthma, in a patient in need thereof.

In an embodiment, the complex is administered in its complexified form to the said patient.

In another embodiment, the constituents of the complex according to the invention, i.e.:
(i) the antibody, or an antigen-binding portion thereof, according to the invention; and
(ii) the IL-2, in particular hIL-2,
are administered separately to the patient, simultaneously, or sequentially, through the same of different routes, the antibody complexing with IL-2 *in vivo.*

In particular, (i) the antibody, or an antigen-binding portion thereof, according to the invention; and (ii) the IL-2, in particular hIL-2, are administered to the patient simultaneously, or sequentially, in particular by the same route.

### Therapeutic implementation according to the invention

As mentioned above, the present invention relates to an antibody, or an antigen-binding portion thereof, according to the invention for its use in the prevention or treatment of respiratory and/or food allergies, or asthma, in a patient in need thereof.

An antibody according to the invention, or an antigen-binding portion thereof, according to the invention, can indeed bind *in vivo* to the IL-2 present in the patient in need thereof and form a complex according to the invention, in particular when said antibody, or an antigen-binding portion thereof, according to the invention is administered to a patient for the treatment of respiratory and/or food allergies, or asthma. Indeed, inflammation already present in said patient as a consequence of the respiratory and/or food allergies, or asthma, induced the *in situ* secretion of IL-2 by immune cells. Said IL-2 is thus available to the antibody, or antigen-binding portion thereof, which is able, as demonstrated in the example, to disseminate systemically in the patient to which it has been administered.

An antibody, antigen-binding portion thereof, or IL-2 complex according to the invention is administered to the patient in a therapeutically effective amount.

By a **"therapeutically effective amount"** of an antibody, antigen-binding portion thereof, or IL-2 complex according to the invention, it is meant a sufficient amount to prevent or treat the disorders considered in the present invention.

It will be understood, however, that the total daily usage of the antibodies, antigen-binding portion thereof, or IL-2 complex according to the invention will be decided by the attending physician within the scope of sound medical judgment. The specific therapeutically effective dose level for any particular patient will depend upon a variety of factors including the disorder being treated and the severity of the disorder; the activity of the specific antibody, antigen-binding portion thereof, or IL-2 complex according to the invention employed; the specific composition employed; the age, body weight, general health, sex and diet of the patient; the time and route of administration, and the rate of excretion of the specific antibody, antigen-binding portion thereof, or IL-2 complex according to the invention; the duration of the treatment; drugs used in combination or coincidental with the specific antibody, antigen-binding portion thereof, or IL-2 complex according to the invention employed; and like factors well known in the medical arts. For example, it is well known within the skill of the art to start doses of the antibody, antigen-binding portion thereof, or IL-2 complex according to the invention at levels lower than those required to achieve the desired therapeutic effect and to gradually increase the dosage until the desired effect is achieved.

In all and any embodiment of the present invention, an antibody, antigen-binding portion thereof, or IL-2 complex according to the invention can be implemented in a composition in a mixture with a pharmaceutically acceptable medium.

As well known in the art, a medium is a "pharmaceutically acceptable medium" if its administration can be tolerated by a recipient patient. Sterile phosphate-buffered saline is one example of a pharmaceutically acceptable carrier. Other suitable carriers are well-known to those in the art. (See, e.g., Gennaro (ed.), Remington's Pharmaceutical Sciences (Mack Publishing Company, 19th ed. 1995).) Formulations may further include one or more excipients, preservatives, solubilizers, buffering agents, albumin to prevent protein loss on vial surfaces, etc.

Pharmaceutical compositions of the invention can be formulated for a topical, oral, parenteral, intranasal, intravenous or intramuscular administration and the like.

Preferably, a pharmaceutical composition contains vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

An antibody, antigen-binding portion thereof, or IL-2 complex according to the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The antibody/portion/complex of the invention may be formulated within a therapeutic composition to comprise about 0.0001 to 1.0 milligrams, or about 0.001 to 0.1 milligrams, or about 0.1 to 1.0 or even about 10 milligrams per dose or so.

Multiple doses can also be administered to a patient in need thereof.

The therapeutic dosing regimen may comprise administering multiple doses of the antibody/portion/complex of the invention, or pharmaceutical composition comprising it, to the subject at a frequency of about once a day, once every two days, once every three days, once every four days, once every five days, once every six days, once a week, once every two weeks, once every three weeks, once every four weeks, once a month, once every two months, once every three months, once every four months, or less frequently.

As mentioned above, the patient in need thereof can be administered with the constituents of a complex of the invention instead of being administered with the complex itself.

In such cases, each previously defined constituent of a complex of the invention is administered to the patient in a separate pharmaceutical composition, through the same or different routes, preferably the same route, sequentially, simultaneously or separatly.

An antibody/portion/complex of the invention, or a pharmaceutical composition, according to the invention, can be administered to the patient in combination with a second therapeutic agent.

As used herein, the phrase **"in combination with"** means that the antibody/portion/complex of the invention, or a pharmaceutical composition of the invention is administered to the patient at the same time as, just before, or just after, administration of the second therapeutic agent.

In a particular embodiment, the second therapeutic agent is present in the pharmaceutical composition comprising an antibody, an antigen-binding portion thereof, or complex according to the invention.

In a related embodiment, the present invention includes the administration of administering a therapeutically effective amount of an antibody, an antigen-binding portion thereof, or complex according to the invention, or of a pharmaceutical composition according to the invention, to a patient who is on a background anti-allergy therapeutic regimen.

The background anti-allergy therapeutic regimen may comprise a course of administration of, for example steroids, antihistamines, decongestants or anti- IgE agents. The antibody, an antigen-binding portion thereof, or complex according to the invention may be added on top of the background anti-allergy therapeutic regimen.

In some embodiments, the antibody, antigen-binding portion thereof, or complex according to the invention is added as part of a "background step-down" scheme, wherein the background anti-allergy therapy is gradually withdrawn from the subject over time while the antibody, an antigen-binding portion thereof, or complex according to the invention is administered to the patient at a constant dose, or at an increasing dose, or at a decreasing dose, over time.

The implementation of an antibody, antigen-binding portion thereof, or complex according to the invention as previously defined also encompasses implementation of an antibody, antigen-binding portion thereof, or complex according to the invention for enhancing the efficacy and/or safety of a desensitization therapy.

Such aspect of the invention comprises administering a therapeutically effective amount of an antibody, antigen-binding portion thereof, or complex according to the invention, or a corresponding pharmaceutical composition, just prior to or concurrent with a desensitization therapy.

A **"desensitization therapy"** refers to the repeated administration of an allergen to a subject over time as means for treating or preventing allergies and allergic reactions, or to reduce or eliminate allergic responses. In a typical desensitization therapy, small amounts of allergen are initially administered to an allergic subject, followed by administration of increased amounts of allergen. In certain instances, the desensitization therapy comprises at least two consecutive phases:
(1) an up-dosing phase, and
(2) a maintenance phase.

In the up-dosing phase, increasing doses of allergen are administered until an effective and safe dose is achieved. The dose that is established at the end of the up-dosing phase is then administered to the subject throughout the course of the maintenance phase. The duration of the up-dosing phase can be several weeks or several months. In certain embodiments, however, the up- dosing phase is of substantially shorter duration, such as less than a week. The maintenance phase of a desensitization therapy most often lasts several months, several years, or indefinitely.

According to this aspect of the invention, the desensitization therapy may comprise administration of a food allergen derived from a food item selected from the group consisting of dairy product, egg, wheat, soy, fish, shellfish, peanut and tree nut. Alternatively, the desensitization therapy may comprise administration of a non-food allergen selected from the group consisting of insect venom, dust, mold, animal dander, pollen, latex, medication, ragweed, grass, and birch, and in particular the administration of an allergen of respiratory allergies, such as one selected from the group consisting of pollen, dust, mould, dust mite debris and hair or animal dander.

An antibody, antigen-binding portion thereof, or complex according to the invention, or a corresponding pharmaceutical composition, can accordingly be administered to the patient throughout the entire course of the desensitization therapy, or for only a portion of the desensitization therapy.

According to the present invention, the efficacy and/or safety of a desensitization therapy is "enhanced" in a subject due to the additional administration of an antibody, an antigen-binding portion thereof, or complex according to the invention, or of a pharmaceutical composition according to the invention when:
- the duration of the up-dosing phase is decreased without compromising efficacy or safety;
- the duration of the maintenance phase is decreased without compromising efficacy or safety;
- the number of doses of allergen administered during the up-dosing or maintenance phase is reduced without compromising efficacy or safety;
- the frequency of allergen administration during the up-dosing or maintenance phase is reduced without compromising efficacy or safety;
- the dose of allergen administered during the up-dosing or maintenance phase is increased without compromising efficacy or safety;
- the frequency of allergic responses or adverse side-effects triggered by the desensitization therapy is reduced or eliminated;
- the use of or need for conventional allergy medications (such as steroids, antihistamines, anti-lgE agents, etc.) is reduced or eliminated during the up-dosing and/or maintenance phases;
- the level of allergen-induced IgE expression is reduced; and/or
- the frequency of anaphylactic reactions is reduced or eliminated.

The efficacy of a desensitization therapy is also of course enhanced if a subject experiences fewer and/or less severe allergic reactions following desensitization therapy in combination with an antibody, an antigen-binding portion thereof, or complex according to the invention, or of a pharmaceutical composition according to the invention than with desensitization therapy alone.

The present invention also relates to a method for the prevention or treatment of respiratory and/or food allergies, or asthma, in a patient in need thereof comprising the administration of an antibody, an antigen-binding portion thereof or a complex according to the invention to the said patient.

The present invention further relates to the

### Kits

Finally, the present invention also relates to a kit for the treatment or prevention of respiratory and/or food allergies in a patient in need thereof, the kit comprising:
- at least one anti-human IL-2 antibody, or an antigen-binding portion thereof, according to the invention; and
- at least one IL-2, in particular at least one human IL-2 (hIL-2).

The invention will be further understood from the following non-limiting examples. The following examples are provided to describe in detail some of the representative, presently preferred methods and materials of the invention. These examples are provided for purposes of illustration of the inventive concepts, and are not intended to limit the scope of the invention as defined by the appended claims.

### [EXAMPLES]

### MATERIALS & METHODS

### Anti-human IL-2 antibody

The anti-human IL-2 antibody according to the invention implemented in the following examples is obtained from the hybridoma deposited at the CNCM under reference CNCM 1-5726.

This monoclonal antibody is characterized in that it comprises a heavy chain wherein the variable domain comprises:
H-CDR1 having an amino acid sequence as set forth in sequence SEQ ID NO: 11;
H-CDR2 having an amino acid sequence as set forth in sequence SEQ ID NO: 12; and
H-CDR3 having an amino acid sequence as set forth in sequence SEQ ID NO: 13;
and a light chain wherein the variable domain comprises:
L-CDR1 having an amino acid sequence as set forth in sequence SEQ ID NO: 16;
L-CDR2 having an amino acid sequence as set forth in sequence SEQ ID NO: 17; and
L-CDR3 having an amino acid sequence as set forth in sequence SEQ ID NO: 18.

In particular, the variable domain of the heavy chain of this antibody has the amino acid sequence set forth as sequence SEQ ID NO: 14 (without leader sequence) or SEQ ID NO: 15 (with leader sequence) and the variable domain of the light chain of this antibody has the amino acid sequence set forth as sequence SEQ ID NO: 19 (without leader sequence) or SEQ ID NO: 20 (with leader sequence).

### Mouse models

In the allergic asthma model, female BALB/cByJ mice (n = 6-8 mice per group) were purchased from Charles River Breeding Laboratories (L'Arbresle, France) and used for all experiments. Mice were housed in a ventilated cage system. Mice were sensitized on days 0, 7, 14 and 21 percutaneously and then challenged intranasally with Dermatophagoides farinae (Der f, Stallergenes Greer, Antony, France) on days 28, 29, 30, 35, 36 and 37 as previously described22. Mice were sacrificed on day 38 for analysis.

Food allergy was modeled with three-week-old female BALB/cJRj mice (Janvier Labs) housed in a ventilated cage system and in specific pathogen-free conditions. After two weeks of acclimatization, mice were sensitized on days 0, 7, 14 and 21 by intraperitoneal injections of 20 µg of deaminated gliadins in 100 µL of phosphate-buffered saline (PBS) plus 100 µL of aluminum hydroxide and challenged twice on days 28 and 35 by oral administration of 20 mg of deaminated gliadins. Control mice were sensitized intraperitoneally with aluminum hydroxide only and challenged with 127 PBS. The ethics committee of Pays de la Loire (CEEA) approved the experiments under accreditation numbers 9456 and 4049.

### Pharmacological study

Spleen cells were collected from 7- to 8-week-old BALB/cByJ-Ly5.1 (Charles River Breeding Laboratories) control mice and used for adoptive transfer. CD4+ T cells were purified by negative selection. Five-week-old female BALB/cJRj (Janvier Labs, Le Genest-Saint-Isle, France) mice were used as recipients. One week after transfer, mice were treated with a complex according to the invention (antibody obtained from the hybridoma deposited at the CNCM under reference CNCM 1-5726 bound to hIL-2) with intraperitoneal injections once a day for five days. Different doses of the complex were injected: 10 times (HD) and 100 times (VHD) higher than the effective dose and 10 times (LD) and 100 times (VLD) lower than the effective dose of 1.5 µg of IL-2.

### Cell culture

*In vitro* activity was carried out in the CTLL-2 cell line (growth dependent on the presence of IL-2). Mouse CD4+ T cells and Tregs were purified from the spleen by negative selection for CD4+ T cells and by positive selection for Tregs (StemCells, Easy SEP). For cell viability and proliferation assays, cells were seeded at 1.10⁴ cells/well for CTLL2 and CD4+ T cells and at 5.10⁴ for Tregs in 96-well plates and cultured for 36 h for CTLL2 and CD4+ T cells and for 96 h for Tregs at 37°C and 5% CO₂. At the end of each time point, the MTT assay (Roche Diagnostics, Meylan France) was performed according to the manufacturer's instructions, and absorbance was measured at 570 nm on a Biotek 800 spectrophotometer (BioTek Instruments SAS).

### Immunoglobulin and enzyme measurements

Allergen-specific IgE was measured in sera of the different groups of mice at the end of the protocol to follow the induction of an allergic response. The production of allergen-specific IgE was assayed in serum samples by indirect ELISA as previously described (Castan L. et al.; Allergy. 2018;73(7):1505-1514). Serum aspartate aminotransferase (AST) and alanine aminotransferase (ALT) activity were assessed using an AST Activity Assay kit and ALT Activity Assay kit (Sigma, Saint-Louis, Missouri, USA) according to the manufacturer's instructions to determine serum AST and ALT levels. A creatinine assay kit and CRP ELISA kit (Sigma, Saint-Louis, Missouri, USA) were used according to the manufacturer's instructions to determine creatinine and CRP levels in serum.

### Flow cytometry

Organs were collected from mice and ground. Cells were transferred to a 96-well plate and stained with different markers. Helios-FITC, Foxp3-PE, CD73-PerCp5.5, Neuropilin-Pe-Cy7, CD39-APC, ICOS-APC-H7, CD4-BV421, CD25-BV510, CD3-FITC, CD122-PE, CD4-PerCp5.5, CCR4-Pe-Cy7, CD8-APC-H7, CD44-BV421, F4/80-FITC, CCR3-PE, Ly6G-PerCp5.5, Ly6C-Pe-Cy7, MHCII-FITC, CD103-PE, CD11b-PerCp5.5, CD11c-BV510, Lineage-FITC, ST2-PE, NKp46-PerCp5.5, CCR6-Pe-Cy7, CD127-APC, CD49b-APC-H7, IL-17-PE, CD4-PerCp5.5, IFN-Pe-Cy7 and IL-4-APC. Cells were analyzed on a Canto II or Fortessa flow cytometer (BD Biosciences). Data were acquired using Diva 8.0 software and analyzed with FlowJoX.

### Airway hyperresponsiveness

AHR was measured in age-matched (11 weeks) BALB/cByJ female mice (n = 6-8 per group) using the forced oscillation technique with a FlexiVent^{®} (SCIREQ Inc., Montreal, Canada) in response to increasing doses of methacholine (0, 10, 15, 20, 40 mg/mL), as previously described in Bouchaud G et al. (The Journal of allergy and clinical immunology. 2015;136(1):197-200 e191). Flexiware^{®} (SCIREQ Inc., Montreal, Canada) software was used for data analysis.

### Lung permeability (VLS)

For the determination of pulmonary wet weight, lungs were weighed before and after lyophilization overnight at 58°C under vacuum according to published protocols in Krieg C et al. (Proceedings of the National Academy of Sciences of the United States of America. 2010;107(26): 11906-11911). Pulmonary wet weight was calculated by subtracting the initial pulmonary weight from the lung weight after lyophilization.

### Histology

Lungs (n = 6-8 mice per group) and guts were fixed in 4% paraformaldehyde for at least 48 h, embedded in paraffin, cut and stained with hematoxylin and eosin for morphologic and inflammatory scoring. The histological score was measured blindly based on bronchial morphology and inflammation (12 points) as previously described in Castan L. et al. (Allergy. 2018 73(7):1505-1514) for the lung. Villus/crypt damage and inflammation in the gut (8 points) were analyzed as previously described in Castan L. et al. (Molecular nutrition & food research. 485 2018;62(17):e1800159).

### Statistics

Data were analyzed using GraphPad Prism 6.0 (La Jolla, CA, USA). Values are expressed as the mean ± SEM and were compared using one-way ANOVA. In the case of a significant ANOVA, means were compared using the Mann-Whitney U test, and the results were corrected by Dunn's multiple-comparisons test. A p-value of less than 0.05 was considered significant.

### Example 1

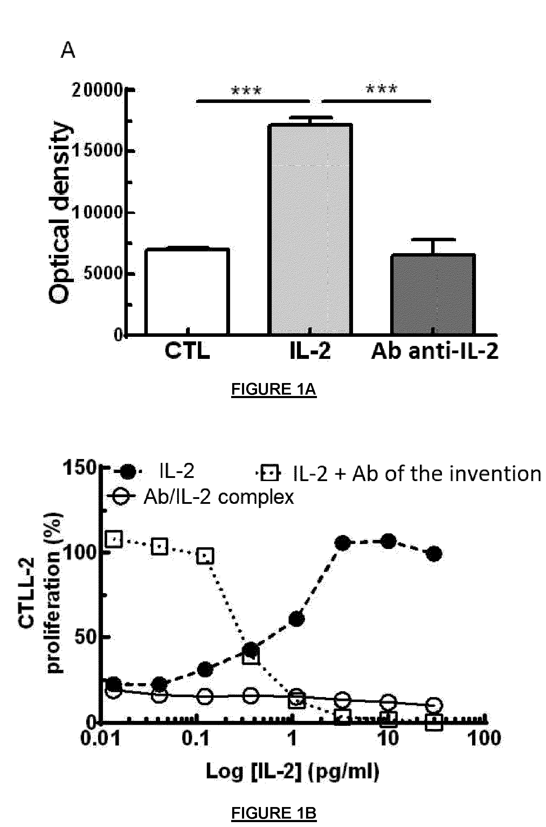

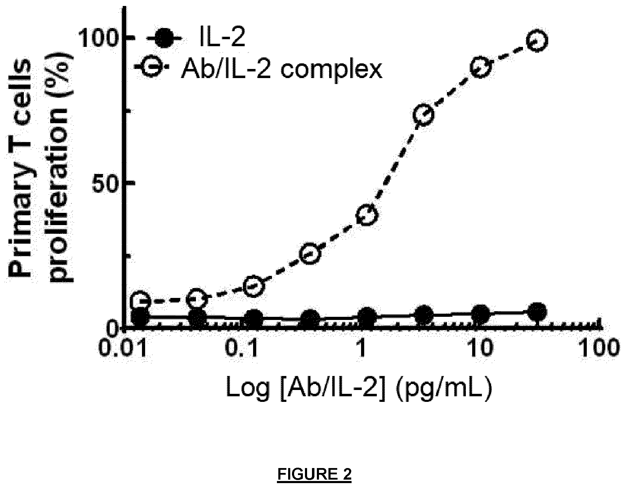

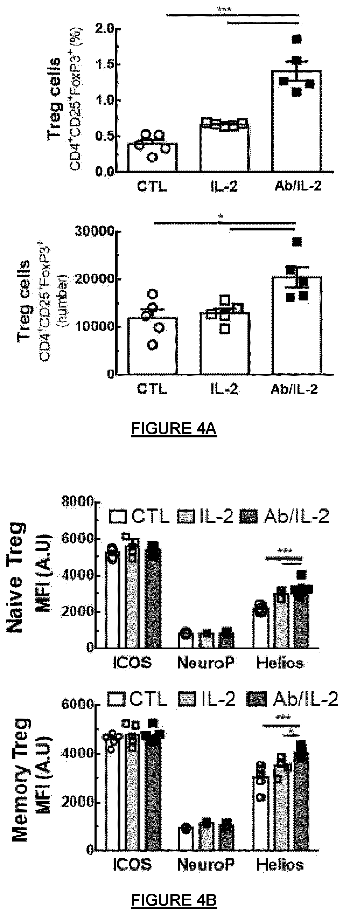

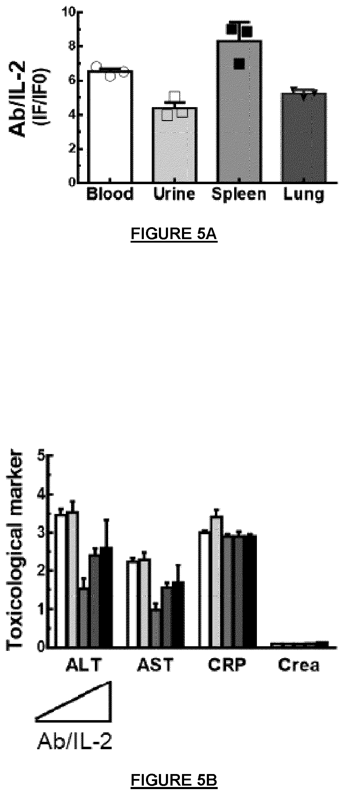

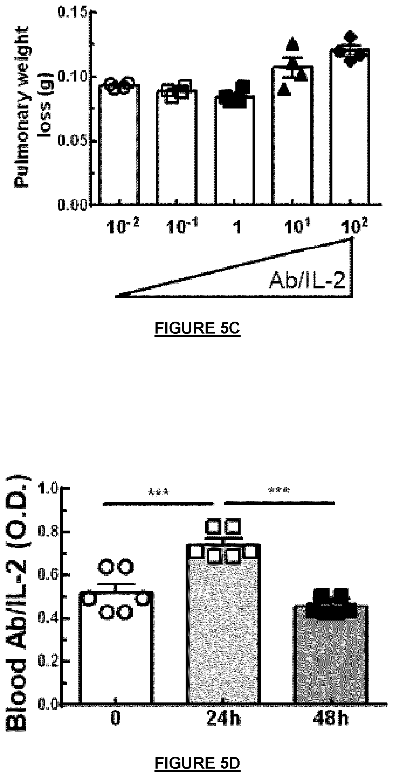

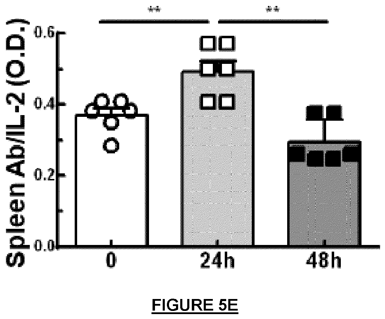

**a.** To evaluate the ability of the above-defined anti-hIL-2 antibody according to the invention to bind IL-2, its binding to hIL-2 via direct ELISA was measured according to well-known methods from the person skilled in the art and as described for example in Arenas-Ramirez N. et al. (Science translational medicine. 2016;8(367):367ra166).
   As expected, optical density increased in wells coated with antibodies of the invention compared to both negative controls, confirming that the anti-hIL-2 antibody of the invention can bind hIL-2 (Figure 1A).
**b.** Then, the biological activity of the complex formed by the antibody of the invention and hIL-2 was assessed. The CTLL-2 cell line, which proliferates in response to hIL-2 and expresses the trimeric receptor of IL-2 composed of CD25 (IL-2Rα), CD122 (IL-2Rβ) and CD132 (γc), was first used. These cells were incubated with increasing concentrations of hIL-2 or fixed hIL-2 concentrations with increasing concentrations of the antibody and increasing concentrations of the complex (Ab/IL-2) (Figure 1B).
   As expected, CTLL-2 cells proliferated in an IL-2 dose-dependent manner. In contrast, CTLL-2 proliferation did not increase with increasing Ab/IL-2 complex concentrations. Moreover, an increased concentration of the antibody inhibited IL-2-induced proliferation (Figure 1B). These results show that the antibody can bind hIL-2 and act as a neutralizing antibody *in vitro.*
**c.** Next, mouse primary CD4+ T cell proliferation was measured.
   IL-2 did not induce CD4+ T cell proliferation at a concentration as high as 30 pg/mL. However, the Ab/IL-2 complex according to the invention was able to induce proliferation in a dose-dependent manner, suggesting a stimulatory activity of the antibody on these cells (Figure 2).
**d.** To measure the ability of the Ab/IL-2 complex to induce T cell proliferation *in vivo,* Ly5.1+ CD4+ T cells labeled with CSFE were adoptively transferred into Ly5.2 mice. Donor Ly5.1+ Treg cell frequencies and numbers were analyzed by flow cytometry in Ly5.2 recipient mice after treatment with the Ab/IL-2 complex at different concentrations.
   The results presented in Figure 3 show an increase in Treg cells in mice treated with the Ab/IL-2 complex at a concentration of 1 (corresponding to a stoichiometric ratio of 1.5 µg of IL-2 and 15 µg of antibody) and higher compared with mice treated ten times less or lower.
**e.** The biological activity of the Ab/IL-2 complex was then characterized *in vivo.*
   Both the ability of IL-2 and the Ab/IL-2 complex to induce Treg cells in mice were measured. To do so, splenic Tregs were analyzed after Ab/IL-2 complex treatment by flow cytometry (Figure 4). Mice treated with the Ab/IL-2 complex displayed an increase in both Treg frequencies and numbers compared to IL-2-treated and control mice (Figure 4A). Spleen naive and memory Treg subsets and the expression of ICOS, Helios and neuropilin-1 (NRP-1) were analyzed on each subset by flow cytometry (Figure 4B).
   Mice treated with PBS or IL-2 did not display a modulation of any T cell subset or their ICOS, Helios and NRP-1 expression. Interestingly, mice treated with the Ab/IL-2 complex displayed an increase in both naive and memory Tregs, and these Treg subsets exhibited an increase in Helios MFI (Figure 4B).
   These results show that the antibody according to the invention potentiates IL-2 to induce Helios+ naive and memory Tregs *in vivo.*
**f.** To determine the possibility of considering an antibody of the invention as a potential biological element, it was determined whether the Ab/IL-2 complex is safe and well tolerated (Figure 5).

To this aim, the biodistribution, toxicological markers and vascular leak syndrome were analyzed after five daily injections of the Ab/IL-2 complex (Figure 5A-C).

The results demonstrate that the Ab/IL-2 complex spreads equally through blood, lungs and urine. The Ab/IL-2 complex concentration was however higher in the spleen, demonstrating a long half-life and a systemic effect (Figure 5A). The measurement of toxicological markers did not highlight differences in blood concentrations of aspartate transaminase (AST), alanine transaminase (ALT), C reactive protein (CRP) or creatinine regarding the Ab/IL-2 complex concentrations (Figure 5B). Finally, as the major dose-limiting toxicity of interleukin-2 (IL-2) and immunotoxin (IT) therapies is vascular leak syndrome (VLS), VLS was also evaluated (Figure 5C) and did not show any increase in pulmonary wet weight at any of the Ab/IL-2 complex concentrations used (Figure 5C).

To determine the clearance of the Ab/IL-2 complex, splenic and blood concentrations were measured at 0, 24 and 48 h after one injection of the complex (Figure 5D and 5E). We observed an increase in the complex 24 h after injection and a decrease to a basal level after 48 h in the spleen and blood of mice (Figure 5D and 5E).

Altogether, the results demonstrate that an Ab/IL-2 complex according to the invention increases Treg cells *in vivo* and is well tolerated, safe and easily eliminated.

### Example 2

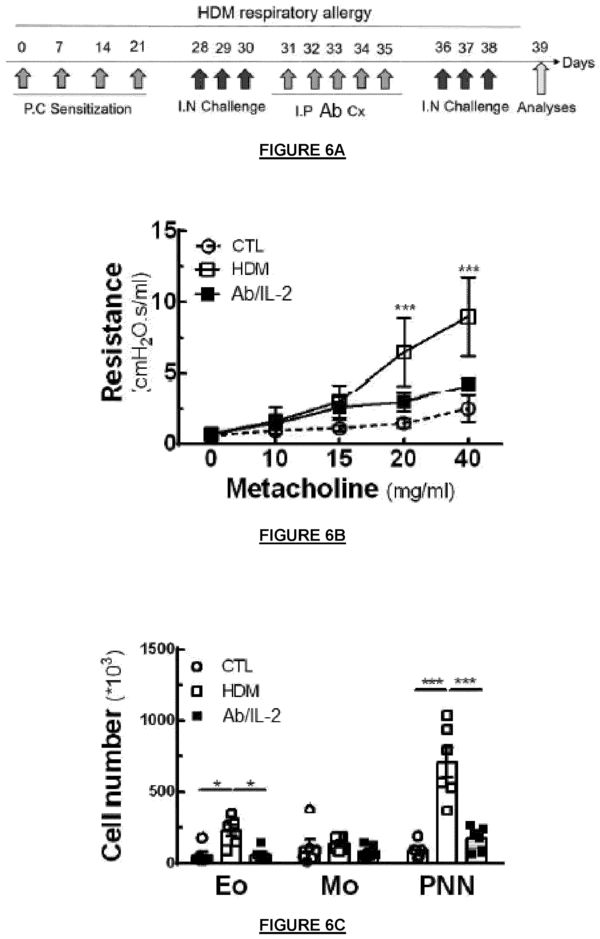

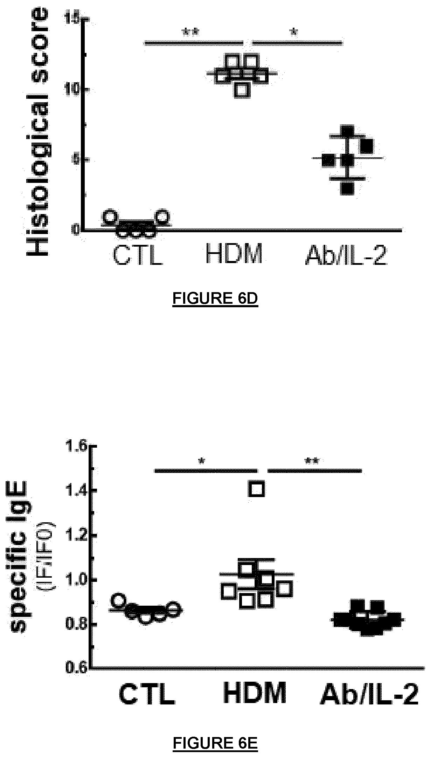

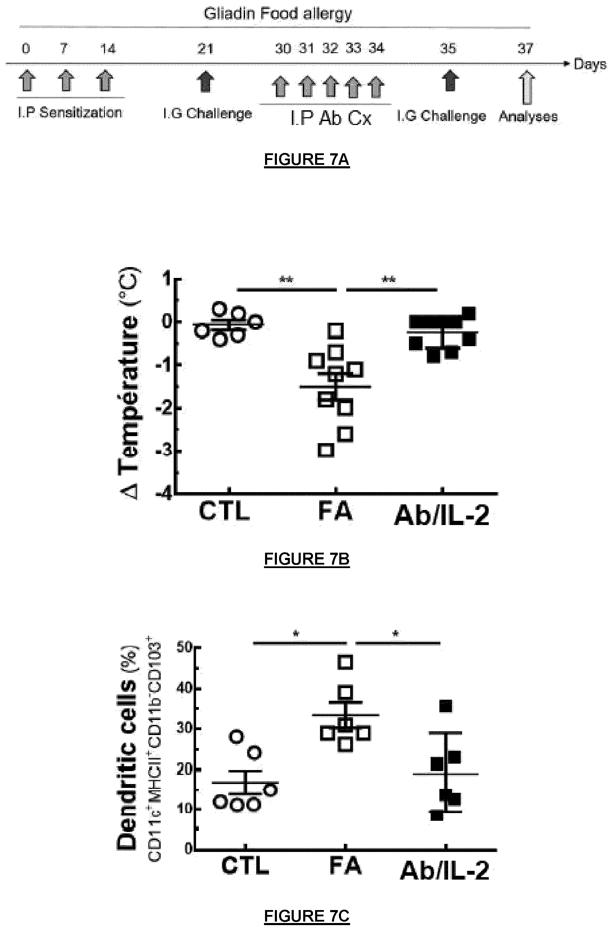

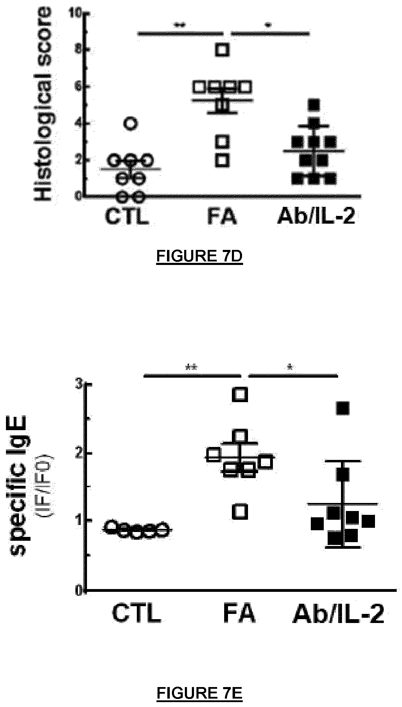

**a.** To investigate the therapeutic potential of an Ab/IL-2 complex according to the invention, its effects were first measured in a mouse model of HDM-induced airway inflammation (Figure 6).
   A characterized model inducing mixed Th2/Th17 inflammation associated with eosinophilic and neutrophilic infiltration was used (Figure 4A) (Chesne J. et al.; The Journal of allergy and clinical immunology. 2015;135(6):1643-1645.E5).
   Mice receiving the Ab/IL-2 complex displayed a decrease in lung resistance in response to methacholine compared to asthmatic mice and a similar level as control mice (Figure 6B). Inflammatory cells from bronchoalveolar lavage (BAL) from mice treated with the Ab/IL-2 complex displayed a strong decrease in eosinophil and neutrophil numbers compared to asthmatic mice (Figure 6C). Subsequently, pulmonary lesions were investigated (Figure 6D). Concordant with lung function measurement and BAL cell numbers, Ab/IL-2 complex-treated asthmatic mice displayed lower perivascular and peribronchial cell infiltration and epithelial cell hyperplasia than asthmatic mice.
   Finally, the effect of Ab/IL-2 complex was explored on circulating HDM-specific IgE as a reflection of the Th2 humoral response (Figure 6E). Although asthmatic mice have a higher level of HDM-specific IgE than control mice, Ab/IL-2 complex-treated mice displayed a level of HDM-specific IgE similar to the control (Figure 6E).
   These results demonstrate that treatment based on the Ab/IL-2 complex according to the invention considerably reduced lung inflammation, AHR and allergen-specific IgE, three cardinal characteristics of allergic asthma.
**b.** The therapeutic potential of an Ab/IL-2 complex of the invention was then investigated in food allergies (Figure 7). Here, again, we used a characterized model of food allergy induced by intraperitoneal sensitization and oral challenges with wheat gliadins (Figure 7A) (Bouchaud G. et al., Journal of agricultural and food chemistry. 2015 ;63(28):6475-6483).
   The effects of the Ab/IL-2 complex on body temperature drop after oral challenge, mesenteric lymph node inflammation and jejunum histology were first measured in a food allergy model. Body temperature was reduced in food allergic mice compared to control mice but not in Ab/IL-2 complex mice (Figure 7B). Then, the implications of CD103+ dendritic cells (CD103+ DCs) was investigated. This DC subset is known to be able to probe food antigens in the gut lumen and migrate to mesenteric lymph nodes (mLNs) to prime naive T cells, leading to Th2 polarization and allergen-specific IgE production.
   Wheat-gliadin-induced food allergic mice displayed an increase in CD103+ DCs. However, this increase was not observed in Ab/IL-2 complex-treated or control mice (Figure 7C).
**c.** Following these results, the effect of the Ab/IL-2 complex was investigated on jejunum tissue. According to body temperature and CD103+ DC measurement, we observed strong structural degradation of the jejunum in food allergic mice, especially on villi and crypts of epithelial cells, compared to controls (Figure 7D). Interestingly, no structural degradation of the jejunum was observed with Ab/IL-2 complex treatment, similar to control mice, and concordant with histological scoring (Figure 7D).
**d.** Finally, the impact of Ab/IL-2 complex on circulating wheat gliadin-specific IgE was also observed. Food allergic mice showed an increase in wheat-gliadin-specific IgE in serum, but control and Ab/IL-2 complex-treated mice did not (Figure 7E).
   Altogether, these results show that an Ab/IL-2 complex according to the invention can abrogate allergic asthma and food allergy features *in vivo* even after allergen sensitization and challenge.

## Claims

1. An isolated anti-human IL-2 antibody, or an isolated antigen-binding portion thereof, comprising:
(i) a heavy chain wherein the variable domain comprises H-CDR1 having an amino acid sequence as set forth in sequence SEQ ID NO: 11; H-CDR2 having an amino acid sequence as set forth in sequence SEQ ID NO: 12 and H-CDR3 having an amino acid sequence as set forth in sequence SEQ ID NO: 13; and
(ii) a light chain wherein the variable domain comprises L-CDR1 having an amino acid sequence as set forth in sequence SEQ ID NO: 16; L-CDR2 having an amino acid sequence as set forth in sequence SEQ ID NO: 17 and L-CDR3 having an amino acid sequence as set forth in sequence SEQ ID NO: 18.

2. The antibody, or antigen-binding portion thereof, according to claim 1, wherein:
- the variable domain of the heavy chain of the antibody has an amino acid sequence having at least 85% sequence identity with an amino acid sequence selected from the sequences set forth as sequence SEQ ID NO: 14 and SEQ ID NO: 15, and a biological activity of the same nature as the sequences set forth as sequence SEQ ID NO: 14 and SEQ ID NO: 15; and/or
- the variable domain of the light chain of the antibody an amino acid sequence having at least 85% sequence identity with an amino acid sequence selected from the sequences set forth as sequence SEQ ID NO: 19 and SEQ ID NO: 20, and a biological activity of the same nature as the sequences set forth as sequence SEQ ID NO: 19 and SEQ ID NO: 20.

3. The antibody, or antigen-binding portion thereof, of claim 1 or 2, said antibody being obtainable from the hybridoma deposited at the CNCM under reference CNCM 1-5726.

4. The antibody, or antigen-binding portion thereof, according to any one of claims 1 to 3, wherein the antibody is a chimeric or humanized antibody.

5. The antibody, or antigen-binding portion thereof, according to any one of claims 1 to 4, wherein the antigen-binding portion is selected from the group consisting of Fab, Fab', F(ab')₂, domain antibodies (dAbs, e.g., shark and camelid antibodies), portions including complementarity determining regions (CDRs), scFv, minibodies, diabodies, triabodies, and tetrabodies.

6. The antibody, or antigen-binding portion thereof, according to any one of claims 1 to 5, wherein the antibody, or antigen-binding portion thereof neutralizes *in vitro* the binding of human IL-2 to the trimeric receptor of IL-2 composed of CD25, CD122 and CD132.

7. An IL-2 complex comprising the anti-human IL-2 antibody, or an antigen-binding portion thereof, according to any one of claims claim 1 to 6, and a human IL-2, the antibody, or antigen-binding portion thereof, being non-covalently bound to the human IL-2.

8. The anti-human IL-2 antibody, or antigen-binding portion thereof, as defined in any one of claims 1 to 6, or the IL-2 complex according to claim 7, wherein the antibody, antigen-binding portion thereof or IL-2 complex induces CD4(+) CD25(+) FoxP3(+) regulatory T cells proliferation *in vivo.*

9. The anti-human IL-2 antibody, or antigen-binding portion thereof, as defined in any one of claims 1 to 6 and 8, for its use in the prevention or treatment of respiratory and/or food allergies, or asthma, in a patient in need thereof.

10. The anti-human IL-2 antibody, or antigen-binding portion thereof, for use according to claim 9, wherein the antibody or antigen-binding portion thereof is administered to the patient in need thereof simultaneously, or sequentially, with human IL-2, in particular by the same route.

11. The anti-human IL-2 antibody, or antigen-binding portion thereof, for use according to claim 10, wherein the IL-2 complex according to claim 8 is administered to the patient in need thereof.

12. The anti-human IL-2 antibody, or antigen-binding portion thereof, for use according to any one of claims 9 to 11, wherein the antibody, antigen-binding portion thereof or IL-2 complex is administered to the patient in need thereof simultaneously, or sequentially, with at least one food and/or respiratory allergen.

13. An isolated nucleic acid encoding the anti-human IL-2 antibody, or an isolated antigen-binding portion thereof, as defined in any one of claims 1 to 6, comprising in particular the nucleic acid sequences SEQ ID NO: 1, SEQ ID NO: 2, SEQ ID NO: 3, SEQ ID NO: 6, SEQ ID NO: 7 and SEQ ID NO: 8, and comprising more particularly the nucleic acid sequences (i) SEQ ID NO: 4 or SEQ ID NO: 5 and (ii) SEQ ID NO: 9 or SEQ ID NO: 10.

14. An expression vector comprising the isolated nucleic acid of claim 13 operably linked to a promoter.

15. A kit for the treatment or prevention of respiratory and/or food allergies in a patient in need thereof, the kit comprising:
- at least one anti-human IL-2 antibody, or an antigen-binding portion thereof, as defined in any one of claims 1 to 6; and
- at least one human IL-2.
